# EUROPEAN PATENT SPECIFICATION

(11) **EP 4 294 576 B1**
(45) Date of publication and mention of the grant of the patent: **25.12.2024**
(21) Application number: 22707971.2
(22) Date of filing: 15.02.2022
(51) Int. Cl.: B03D 1/008, B03D 1/01, B03D 1/012, B03D 1/014, B03D 1/018, B03D 1/02, C07C 333/14

(54) **DITHIOCARBAMATE DEPRESSANTS, METHODS AND USES THEREOF IN FROTH FLOTATION MINERAL SEPARATION**
DITHIOCARBAMAT-DRÜCKER, VERFAHREN UND VERWENDUNGEN DAVON IN DER MINERALTRENNUNG DURCH SCHAUMFLOTATION
DÉPRIMANTS DE DITHIOCARBAMATE, LEURS PROCÉDÉS ET UTILISATIONS DANS LA SÉPARATION DE MINÉRAUX PAR FLOTTATION PAR MOUSSAGE

(30) Priority: 17.02.2021 EP 21157680
(43) Date of publication of application: 27.12.2023
(73) Proprietor: Tessenderlo Kerley, Inc., Phoenix, AZ 85018 (US)
(72) Inventor: RHODEHOUSE, Melissa, Phoenix, AZ 85018 (US); VASUDEVAN, Mukund, Phoenix, AZ 85018 (US); HOJJATIE, Michael, Phoenix, AZ 85018 (US); MORAN, Paul, Phoenix, AZ 85018 (US)
(74) Representative: Nederlandsch Octrooibureau
(86) International application number: PCT/US2022/016389
(87) International publication number: WO 2022/177872

(56) References cited:
- US-A- 4 990 656
- US-A1- 2018 099 293
- US-A1- 2019 233 372
- DATABASE WPI Week 199135, Derwent World Patents Index; AN 1991-258316, XP002803662

## Description

### FIELD OF THE INVENTION

The invention relates to dithiocarbamic acids or salts thereof and their use in the recovery of minerals, for example as a depressant in froth flotation separation of minerals, in particular separation of molybdenum sulfides from copper and/or iron sulfides. The invention further relates to a froth flotation mineral recovery process employing said dithiocarbamic acids or salts thereof. The invention further relates to compositions comprising said dithiocarbamic acids or salts thereof and kits of parts comprising said compositions.

### BACKGROUND OF THE INVENTION

Froth flotation is a well-known separation technique employed in the field of mineral processing to separate gangue material from valuable minerals, thereby obtaining a pulp comprising the minerals of interest (often referred to as "mineral concentrate"). Froth flotation relies on hydrophobicity differences between valuable minerals and waste gangue to achieve their separation. While some minerals are naturally hydrophobic and accumulate in the froth, a collector is typically added to increase the affinity of the desired minerals to the froth. Froth flotation separation is often applied in the form of a multi-stage process, the actual number of froth flotation stages depending on factors such as the ore being processed, the desired minerals, the desired yield, the desired purity, etc.

In case complex (mixed) ores (such as chalcopyrite-containing ores) are being processed, froth flotation is also used to further process the mineral concentrate obtained from gangue separation in order to (selectively) separate different minerals, relying on hydrophobicity differences between different minerals to achieve their separation. The technique is used for the separation of a large range of sulfides, carbonates and oxides prior to further refinement. Notable sulfide mineral separations are Copper-Molybdenum, Lead-Zinc, Gold-Silver and Nickel-Copper. In order to steer hydrophobicity differences between minerals and achieve such selective mineral separation, a depressant is typically added. The depressant selectively decreases the affinity of some minerals to the froth such that they remain depressed in the bulk pulp.

Existing froth flotation mineral separation practices generally utilize depressants that lead to concerns with respect to health, safety, and environmental issues. For example, for the separation of molybdenum sulfides from copper sulfides, current practices in the industry consist of a number of different chemical schemes which use Sodium hydrosulfide (NaHS or NaHS), Ferrocyanides, or Nokes reagent (a blend of thiophosphates or dithioarsenates and usually also containing sulfides). These raise many concerns, including transporting materials that contain or readily form hydrogen sulfide, utilizing reagents in the flotation process that are highly toxic and/or that in use form highly toxic hydrogen sulfide off-gassing, environmental impact in case of tailings reservoir failure etc. A more detailed description of background art in the field of Cu-Mo separation can be found in WO2015157498 paragraphs 2-11, incorporated herein by reference.

Some alternative reagents have emerged recently for use as a depressant in mineral separation processes, these may also have drawbacks, such as decreased performance compared to NaHS, poor availability, high cost, toxicity, environmental concerns, low efficacy such that many flotation stages are required, non-selective depression such that no separation of different valuable minerals can be achieved, etc.

For example, US4595538 describes the use of Tri-alkali metal di(carboxyalkyl) dithiocarbamates and triammonium-di(carboxyalkyl) dithiocarbamates as depressants.

All in all, any improvements in mineral flotation practice, especially with respect to health and safety, would be of significant importance.

Hence, there remains a need for alternative depressants for use in the froth flotation separation of minerals, in particular for the separation of metal sulfides, for example the depression of copper and iron sulfides in molybdenite flotation circuits.

### SUMMARY OF THE INVENTION

The present inventors have identified a distinct class of dithiocarbamic acid or salt thereof depressants, useful in the froth flotation separation of minerals, in particular for the depression of copper and iron sulfides in molybdenite flotation circuits.

The invention is defined by the appended set of claims. Hence, in a first aspect the invention concerns a process for recovering a first mineral comprising the steps of:
(a) providing a pulp comprising solids and water, wherein the solids comprise the first mineral;
(b) adding a first depressant to the pulp;
(c) subjecting the pulp to a froth flotation process to produce a froth comprising the first mineral; and
(d) recovering the froth;
wherein the first mineral is a sulfide mineral, and the first depressant consists of one or more dithiocarbamic acids or salts thereof of formula (I) 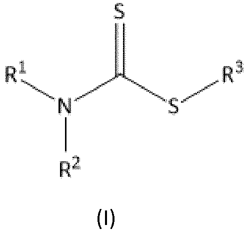 wherein
R¹ represents a first substituent having from 1 to 8 carbon atoms and comprising at least one functional group selected from alcohols, amines, ethers, ketones, acetals, ketals, aminoacetals, hemiaminal ethers or combinations thereof, preferably selected from alcohols, amines, ethers or combinations thereof, wherein R¹ comprises at most two amine functional groups;
   and
R² represents H or a second substituent having from 1 to 8 carbon atoms and optionally comprising at least one functional group selected from alcohols, amines, ethers, ketones, acetals, ketals, aminoacetals, hemiaminal ethers or combinations thereof, preferably selected from alcohols, amines, ethers or combinations thereof;
   or
R¹ and R² are connected to form a heterocyclic 3 to 7 membered ring comprising at least 2 heteroatoms, which is optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from H and C₁-C₄ alkyl;
   and
R³ represents hydrogen or a cation.
with the provisio that when R¹ comprises no other functional groups than alcohols, the process does not comprise the use of a second depressant selected from polymers comprising an allyl thiourea functional group and a hydrophilic acrylamide group;
characterized in that recovering the froth in step (d) provides a second pulp comprising the first mineral which has an increased concentration of the first mineral based on dry weight, compared to the pulp provided in step (a).

US4990656 provides a polyamine substituted dithiocarbamate represented by the formula (R₁R₂N-R₃-NH-CS2)ₓM
wherein R₁ and R₂ are each an aminohydrocarbyl group, R₃ is a hydrocarbyl group, M is selected from the group consisting of an alkali metal and an alkaline earth metal, and x is 1
or 2. The compound is useful as a minerals depressant in an ore flotation process.

SU1614853A1 describes the use of a *N*-(2-Aminoethyl)carbamodithioic acid salt to depress the sulfide minerals galena (PbS), sphalerite ((Zn,Fe)S) and barite (BaSO4) in order to obtain valuable non-sulfide minerals in the foam.

US2019/0336984A1 describes the use of specific dithiocarbamates to improve the depressant performance of polymers comprising an allyl thiourea functional group and a hydrophilic acrylamide group.

Formula (I) defines a limited group of dithiocarbamic acids or salts thereof wherein at least one of the N substituents comprises a functional group selected from alcohols, amines, ethers, ketones, acetals, ketals, aminoacetals, hemiaminal ethers or combinations thereof. As is illustrated in the appended examples, the present inventors have surprisingly found that dithiocarbamic acids or salts thereof according to formula (I) can be used to achieve highly efficient selective mineral depression and are stable in the operating conditions of a froth flotation cell. In particular, it was found that the compounds of formula (I) can be used to separate different sulfide minerals by selectively depressing one or more sulfide minerals, while allowing one or more other sulfide minerals to be collected in the froth. Furthermore, it was surprisingly found that the compounds of formula (I) can be used effectively without requiring a second depressant.

Another aspect of the invention concerns a process for recovering a first mineral, said process comprising the steps of:
(a) providing a pulp comprising solids and water, wherein the solids comprise the first mineral;
(b) adding a first depressant to the pulp;
(c) subjecting the pulp to a froth flotation process to produce a froth comprising the first mineral; and
(d) recovering the froth;
characterized in that the first mineral is a sulfide mineral, and the first depressant consists of one or more dithiocarbamic acids or salts thereof of formula (I) as described herein,
wherein
R¹ represents a first substituent having from 1 to 8 carbon atoms and comprising at least one functional group selected from alcohols, amines, ethers, ketones, acetals, ketals, aminoacetals, hemiaminal ethers or combinations thereof, preferably selected from alcohols, amines, ethers or combinations thereof, wherein R¹ comprises at most two amine functional groups;
   and
R² represents H or a second substituent having from 1 to 8 carbon atoms and optionally comprising at least one functional group selected from alcohols, amines, ethers, ketones, acetals, ketals, aminoacetals, hemiaminal ethers or combinations thereof, preferably selected from alcohols, amines, ethers or combinations thereof;
   or
R¹ and R² are connected to form a heterocyclic 3 to 7 membered ring comprising at least 2 heteroatoms, which is optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from Hand C₁-C₄ alkyl;
   and
R³ represents hydrogen or a cation;
with the provisio that when R¹ comprises no other functional groups than alcohols, the process does not comprise the use of a second depressant selected from polymers comprising an allyl thiourea functional group and a hydrophilic acrylamide group;
with the provisio that the first depressant is not N-(2-aminoethyl)dithiocarbamic acid or a salt thereof.

In another aspect, the invention concerns a composition comprising the first depressant as described in claim 1 of the appended set of claims and further comprising a sulfide mineral, preferably a sulfide mineral selected from the group consisting of Molybdenite, Chalcopyrite, Chalcocite, Covellite, Pyrite, Bornite, Sphalerite, and combinations thereof, preferably selected from the group consisting of Molybdenite and Chalcopyrite;
with the provisio that the first depressant is not *N*-(2-Aminoethyl)carbamodithioic acid or a salt thereof or a compound of formula (I) wherein R¹ comprises no other functional groups than alcohols.

In another aspect the invention concerns a composition comprising a first and further comprising one, two, or three of:
- a collector;
- a second depressant; and
- a mineral;

wherein the first depressant consists of one or more dithiocarbamic acids or salts thereof of formula (I) as described in claim 1 of the appended set of claims,
wherein
   R¹ represents a first substituent having from 2 to 5 carbon atoms and comprising one or two alcohols; or
   R¹ and R² are identical; or
   R¹ and R² are connected to form a heterocyclic 5 or 6 membered ring comprising 2 heteroatoms selected from N and O, which is optionally substituted with a single functional group selected from - C₁-C₄ alkyl, preferably the heterocyclic ring is substituted with a single methyl group; or
   the dithiocarbamic acids or salts thereof of formula (I) are selected from the group consisting of compounds 1,3-18, 20 acid as defined in claim 8 or a salt thereof,
with the provisio that the first depressant is not a compound of formula (I) wherein R¹ comprises no other functional groups than alcohols.

In another aspect the invention concerns the use of a dithiocarbamic acid or salt thereof of formula (I) as described herein in the recovery of minerals, preferably the froth flotation recovery, of minerals with the provisio that the dithiocarbamic acid or salt thereof is not *N*-(2-Aminoethyl)carbamodithioic acid or a salt thereof or a compound of formula (I) wherein R¹ comprises no other functional groups than alcohols.

Part of the disclosure, but not of the claims, concerns a kit of parts comprising a composition (A) comprising the first depressant as described herein and instructions for use of the composition (A) as a depressant in froth flotation recovery of minerals.

Another part of the disclosure, but not of the claims, concerns a kit of parts comprising
- a composition (A) comprising the first depressant as described herein;
- a composition (B) comprising a second depressant; and
- optionally instructions for use of the composition (A) as a depressant in froth flotation recovery of minerals.

In another aspect the invention concerns a dithiocarbamic acid or salt thereof which is 3-amino-1,2-propanediol dithiocarbamic acid or a salt thereof, 2-amino-2-methyl-1,3,-propanediol dithiocarbamic acid or a salt thereof, or *N*-phenylethylenediamine dithiocarbamic acid or a salt thereof.

Another part of the disclosure, but not the claims, concerns a method of synthesizing a dithiocarbamic acid or salt thereof of formula (I) as described herein, comprising the steps of:
(i) providing an amine of formula R¹R²NH;
(ii) providing CS₂; and
(iii) reacting the amine of step (i) with the CS₂ of step (ii) under suitable conditions to form the dithiocarbamic acid or salt thereof of formula (I);
wherein optionally a base, such as KOH or NaOH, preferably NaOH is added
- to the amine of step (i) before step (iii);
- to the CS₂ of step (ii) before step (iii); and/or
- to the reaction product of step (iii).

### DETAILED DESCRIPTION OF THE INVENTION

### The dithiocarbamic acids or salts thereof of formula (I) used in different aspects of the invention

The different aspects of the present invention as set out herein are based on the finding that a distinct class of dithiocarbamic acids or salts thereof, namely dithiocarbamic acids or salts thereof of formula (I), have specific properties rendering them useful as depressants in froth flotation mineral recovery. Hence, the identity of these dithiocarbamic acids or salts thereof of formula (I) and preferred embodiments thereof will first be discussed. The embodiments described herein in relation to the dithiocarbamic acids or salts thereof of formula (I) are applicable to all aspects of the invention, for example to their use in a froth flotation process according to the invention, to the formulations according to the invention comprising a dithiocarbamic acid or salt thereof of formula (I), to the uses according to the invention of the dithiocarbamic acids or salts thereof of formula (I) and to the kit of parts according to the disclosure, comprising a dithiocarbamic acid or salt thereof of formula (I).

It will be understood by the skilled person, in light of the present disclosure, that the expression "a substituent having from 1 to 8 carbon atoms and comprising at least one functional group" or similar expressions as used herein should be construed to specify the total amount of carbon atoms inclusive of any carbon atoms brought by any functional groups comprised in the substituent. For example, even if R¹ or R² comprises an amine (which may be secondary or tertiary) or ether functional group, this implies that the whole of R¹ contains 1 to 8 carbon atoms.

It will also be understood by the skilled person that, when defining chemical substituents throughout this specification, such as in the context of R¹ and R² of formula (I), definition of a substituent such as an alkyl chain is intended to encompass linear and branched forms falling within the definition of that substituent, unless indicated otherwise.

It will also be understood by the skilled person that, in accordance with the invention, the definition for R¹ and R² is a closed definition and should be interpreted to mean that R¹ and R² do not comprise any other functional groups than the recited functional groups.

It will also be understood by the skilled person, based on the present teachings, that the compounds of the invention can be provided in free acid form, in the form of a salt, typically a base addition salt, in the form of a mixture of different salts or in the form of a mixture of the free acid and one or more salt forms. In embodiments of the invention R³ represents hydrogen. In embodiments of the invention R³ represents a cation. In principle, the invention is not limited to any specific (group) of salts and R³ can represent any organic or inorganic cation. In certain embodiments, it may be preferable that the cation is selected from the group consisting of alkaline metal ions, alkaline earth metal ions and quaternary ammonium cations represented by the formula NRR'R"R‴ wherein R, R', R" and R'" are independently selected from the group consisting of C₁-C₆ alkyl and C1 -C₆ hydroxyalkyl, more preferably the cation is selected from the group consisting of alkaline metal ions and alkaline earth metal ions, more preferably the cation is sodium, calcium, magnesium and/or potassium, most preferably sodium.

In embodiments of the invention, R¹ represents a first substituent having from 1 to 6 carbon atoms, preferably from 2 to 5 carbon atoms, and comprising at least one functional group selected from alcohols, amines, ethers, ketones, acetals, ketals, aminoacetals, hemiaminal ethers or combinations thereof, preferably selected from alcohols, amines, ethers or combinations thereof. Preferably R¹ comprises one or two functional groups selected from alcohols, amines, ethers, ketones, acetals, ketals, aminoacetals, hemiaminal ethers or combinations thereof, preferably selected from alcohols, amines, ethers or combinations thereof. Hence, in highly preferred embodiments of the invention, R¹ represents a first substituent having from 2 to 5 carbon atoms and comprising one or two functional groups selected from alcohols, amines, ethers or combinations thereof, more preferably comprising one or two functional groups selected from amines, ethers or combinations thereof. Examples of such preferred embodiments are compounds of formula (I) wherein:
- R¹ represents a first substituent having from 2 to 5 carbon atoms and comprising one or two alcohols; or
- R¹ represents a first substituent having from 2 to 5 carbon atoms a comprising one secondary or tertiary amine, preferably R¹ represents a first substituent having from 4 to 5 carbon atoms and comprising one tertiary amine.

In some embodiments of the invention, R¹ and R² are identical. Examples of such embodiments are compounds of formula (I) wherein R¹ and R² are identical and wherein:
- R¹ represents a first substituent having from 2 to 5 carbon atoms and comprising one alcohol; or
- R¹ represents a first substituent having from 4 to 5 carbon atoms and comprising one tertiary amine.

In some embodiments of the invention, R¹ and R² are not identical. In such embodiments it is preferred that R² represents H, phenyl or a C₁-C₅ alkyl, preferably R² represents H, phenyl or a C₁-C₂ alkyl, most preferably R² represents H or a C₁-C₂ alkyl. Examples of such preferred embodiments are compounds of formula (I) wherein R² represents H, phenyl or a C₁-C₅ alkyl, preferably R² represents H, phenyl or a C₁-C₂ alkyl, and wherein:
- R¹ represents a first substituent having from 2 to 5 carbon atoms and comprising one or two alcohols; or
- R¹ represents a first substituent having from 2 to 5 carbon atoms a comprising one secondary or tertiary amine, preferably R¹ represents a first substituent having from 4 to 5 carbon atoms and comprising one tertiary amine.

For all embodiments described herein wherein R¹ represents a first substituent having from 2 to 5 carbon atoms and comprising one or two alcohols, a particular embodiment of the process described herein is provided wherein the process does not comprise the use of a second depressant selected from polymers comprising an allyl thiourea functional group and a hydrophilic acrylamide group, more preferably wherein the process does not comprise the use of any other depressant than the first depressant.

In all embodiments R¹ comprises at most two amine functional groups. As will be understood by the skilled person, this implies that R¹ is not a polyamine substituted dithiocarbamate. It is even more preferred that R¹ comprises at most two functional groups of the same type, a type being selected from alcohols, amines, ethers, ketones, acetals, ketals, aminoacetals, hemiaminal ethers or combinations thereof.

Furthermore, according to highly preferred embodiments of the invention all carbon atoms comprised in R¹ and R² are saturated. In particularly preferred embodiments of the compounds of formula (I), the examples described in the previous paragraphs are provided wherein R¹ and R² are saturated. The skilled person will understand, in light of the present disclosure, that embodiments of R¹ or R² wherein "all carbon atoms are saturated" or similar expressions as used herein imply that all carbon atoms in R¹ or R² are sp³ hybridized meaning that they are not connected via double or triple bonds. Hence, such embodiments are identical to expressing that R¹ or R² consist of an alkyl chain wherein optionally one or more carbon atoms have been substituted by heteroatoms in order to provide the functional groups as defined herein for R¹ and R², and these expressions may be used interchangeably.

In embodiments of the invention the compound of formula (I) is such that R¹ represents -R⁴-OH, -R⁵-O-R⁶, or -R⁷NR⁸R⁹
wherein
R⁴ is selected from the group consisting of C₁-C₆ alkyl, C₂-C₆ alkenyl, C₂-C₆ alkynyl, and C₂-C₆ hydroxyalkyl, preferably from the group consisting of C₁-C₆ alkyl and C₂-C₆ hydroxyalkyl, more preferably from the group consisting of C₂-C₃ alkyl and C₃-C₄ hydroxyalkyl;
R⁵ is selected from the group consisting of C₁-C₆ alkyl, C₂-C₆ alkenyl, C₂-C₆ alkynyl, and C₂-C₆ hydroxyalkyl, preferably from the group consisting of C₁-C₆ alkyl, more preferably from the group consisting of C₂-C₃ alkyl;
R⁶ is selected from the group consisting of C₁-C₆ alkyl, C₂-C₆ alkenyl, C₂-C₆ alkynyl, and C₂-C₆ hydroxyalkyl, preferably from the group consisting of C₁-C₆ alkyl, more preferably from the group consisting of C₁-C₂ alkyl;
R⁷ is selected from the group consisting of C₁-C₆ alkyl, C₂-C₆ alkenyl, C₂-C₆ alkynyl, and C₂-C₆ hydroxyalkyl, preferably from the group consisting of C₁-C₆ alkyl, more preferably from the group consisting of C₂-C₃ alkyl; and
R⁸ and R⁹ are independently selected from the group consisting of H, C₁-C₆ alkyl, C₂-C₆ alkenyl, C₂-C₆ alkynyl, and C₂-C₆ hydroxyalkyl, preferably from the group consisting of H and C₁-C₆ alkyl more preferably from the group consisting of H and methyl;
and wherein R² represents H, C₁-C₅ alkyl, -R⁴-OH, -R⁵-O-R⁶, or -R⁷NR⁸R⁹, preferably R² represents H, C₁-C₂ alkyl or a functional group identical to R¹.

In some embodiments R¹ and R² are connected to form a heterocyclic 3 to 7 membered ring comprising at least 2 heteroatoms, which is optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from H and C₁-C₄ alkyl. The heteroatoms comprised in the heterocyclic 3 to 7 membered ring are preferably independently selected from S, N, and O, more preferably they are independently selected from N and O. The heterocyclic ring is preferably 5 or 6 membered. The heterocyclic ring preferably comprises 2 hetero atoms.

Hence, in preferred embodiments R¹ and R² are connected to form a heterocyclic 5 or 6 membered ring comprising two heteroatoms which are N and O (e.g. a morpholine ring) or which are two N atoms (e.g. an imidazole ring), the heterocyclic ring being optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from H and C₁-C₄ alkyl. Examples of such preferred embodiments are compounds of formula (I) wherein R¹ and R² are connected to form a heterocyclic 5 or 6 membered ring comprising two heteroatoms which are N and O or which are two N atoms, the heterocyclic ring being optionally substituted with one or two, preferably one functional groups selected from -OH and C₁-C₄ alkyl, preferably selected from -OH and -CH₃, more preferably selected from -CH₃.

Highly preferred compounds of formula (I) are the dithiocarbamic acids or salts thereof defined in the following table. Even more preferred compounds of formula (I) are compound 1, 3-6, 8-10, 12-20 acid as defined in the following table, or a salt thereof. Most preferred compounds of formula (I) are compound 1, 3, 8, 9 acid as defined in the following table, or a salt thereof.

| **Compound name** | **Compound Structure** | **CAS** | **Systematic name** |
|---|---|---|---|
| diethanolamine dithiocarbamic acid (c**ompound 1 acid**) | 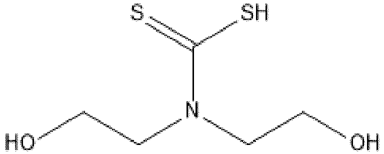 | 1528-72-9 | Bis(2-hydroxyethyl)carbam odithioic acid |
| 3-amino-1,2-propanediol dithiocarbamic acid (**compound 3 acid**) | 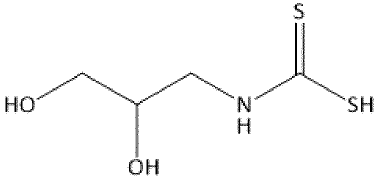 | NA | 2,3-dihydroxypropylcarb amodithioic acid |
| 2-(methylamino)etha nol dithiocarbamic acid (**compound 4 acid**) | 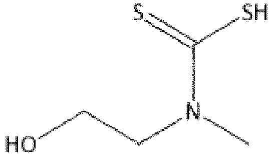 | 91308-51-9 | methyl(2-hydroxyethyl)carbam odithioic acid |
| bis(2-hydroxypropyl)ami ne dithiocarbamic acid (**compound 5 acid**) | 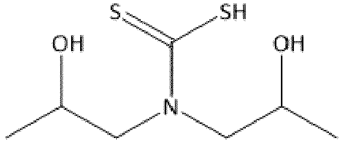 | 144796-43-0 | Bis(2-hydroxypropyl)carba modithioic acid |
| bis(2-methoxyethyl)amin e dithiocarbamic acid (**compound 6 acid**) | 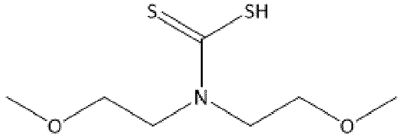 | 395653-20-0 | Bis(2-methoxyethyl)carba modithioic acid |
| 2-amino-2-methyl-1,3,-propanediol dithiocarbamic acid (**compound 7 acid**) | 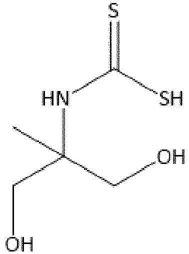 | NA | 2-amino-2-methyl-1,3,-propanediol carbamodithioic acid |
| morpholine dithiocarbamic acid (**compound 8 acid**) | 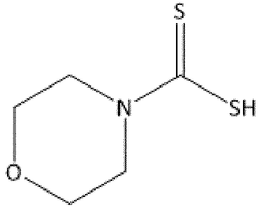 | 3581-30-4 | 4-Morpholinecarbodit hioic acid |
| N-methylpiperazine dithiocarbamic acid (**compound 9 acid**) | 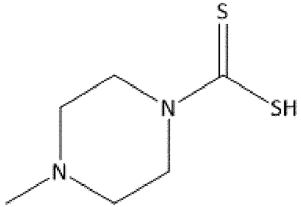 | 5430-77-3 | 4-Methyl-1-piperazinecarbodithi oic acid |
| N-(2-hydroxyethyl)anilin e dithiocarbamic acid (**compound 10 acid**) | 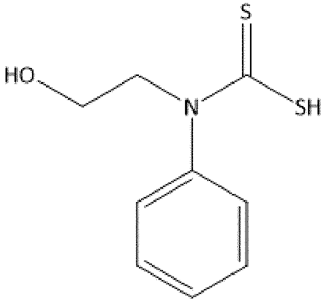 | 1006655-77-1 | phenyl(2-hydroxyethyl)carbam odithioic acid |
| N-phenylethylenedia mine dithiocarbamic acid (**compound 11 acid**) | 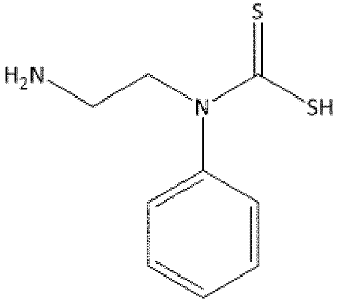 | NA | phenyl(2-aminoethyl)carbamo dithioic acid |
| (2-methoxyethyl)meth ylamine dithiocarbamic acid (**compound 12 acid**) | 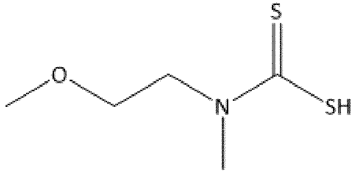 | 884739-17-7 | methyl(2-methoxyethyl)carba modithioic acid |
| 3,3'-iminobis(N,N-dimethylpropylami ne) dithiocarbamic acid (**compound 13 acid**) | 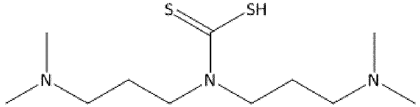 | 1161069-75-5 | *Bis*[(3-(dimethylamino)pro pyl]carbamodithioic acid |
| 2-(ethylamino)ethan ol dithiocarbamic acid (**compound 14 acid**) | 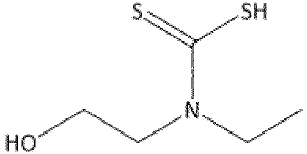 | 91308-52-0 | Ethyl(2-hydroxyethyl)carbam odithioic acid |
| 2-(isopropylamino)et hanol dithiocarbamic acid (**compound 15 acid**) | 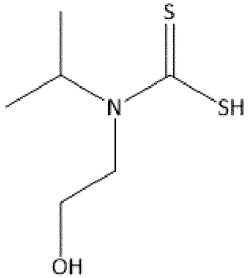 | 1442373-54-7 | isopropyl(2-hydroxyethyl)carbam odithioic acid |
| ethanolamine dithiocarbamic acid (**compound 16 acid**) | 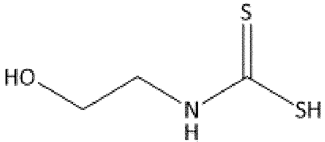 | 59333-68-5 | 2-hydroxyethylcarbam odithioic acid |
| sodium N,N'-Dimethylethylenedi amine dithiocarbamic acid (**compound 17 acid**) | 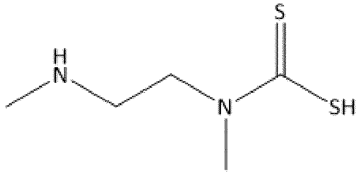 | 58708-60-4 | methyl (2-N-methylaminoethyl)carbamo dithioic acid |
| piperazine dithiocarbamic acid (**compound 18 acid**) | 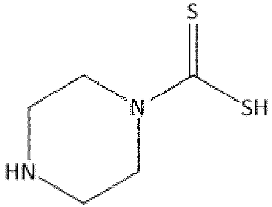 | 99-00-3 | 1-Piperazinecarbodithi oic acid |
| ethylenediamine dithiocarbamic acid (**compound 19 acid**) | 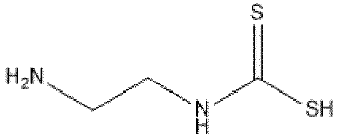 | 20950-84-9 | (2-Aminoethyl)carbamo dithioic acid |
| imidazole dithiocarbamic acid (**compound 20 acid**) | 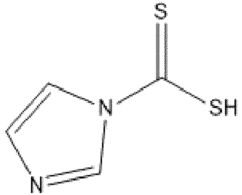 | 732930-06-2 | 1H-Imidazole-1-carbodithioic acid |

In particular embodiments of the invention, the first depressant is as described above, with the provisio that the compound of formula (I) is not *N*-(2-Aminoethyl)carbamodithioic acid or a salt thereof.

### The froth flotation process of the invention

Froth flotation processes in general are known to the skilled person and it is within the routine capabilities of the skilled person, in light of the present disclosure, to adjust operating parameters such that an efficient separation is achieved when operating a process according to the present invention, taking into account factors such as the ore being processed, the desired minerals, the desired yield, the desired purity, the mineral concentration of the pulp, the particle size of the minerals, the water hardness, etc. Reference can be made in this regard to the handbook Fuerstenau, M. C., Jameson, G. J., & Yoon, R. H. (Eds.). (2007). Froth flotation: a century of innovation. SM E.

The pulp provided in step (a) of the process according to the invention is also referred to interchangeably as a "slurry". In preferred embodiments of the invention, the pulp provided in step (a) is a mineral concentrate obtained from the separation of minerals from gangue material in a previous froth flotation stage.

Since transport cost of non-refined ores is not justified by their value, a mining site typically incorporates all stages of ore refinement on-site, starting from mining an ore, followed by comminuting the ore to liberate minerals contained therein and preparing a pulp for froth flotation separation of gangue material. Hence, in some embodiments of the invention step (a) comprises the steps of:
(a1) providing an ore;
(a2) comminuting the ore to obtain comminuted ore having a particle size P80 of less than 500 µm, preferably less than 100 µm
(a3) subjecting the comminuted ore to a froth flotation process to at least partially remove gangue material from the ore, thereby obtaining a pulp comprising solids and water, wherein the solids comprise the first mineral.

Comminution is typically performed via milling and/or breakage comminution devices, such as autogenous (AG) milling, semiautogenous (SAG) milling, ball milling, rod milling, high pressure grinding rolling (HPGR), vertical roller milling (VRM) etc. Classification devices, such as a rake classifier or cyclones are usually employed in combination with grinding to obtain a comminuted ore having the desired particle size distribution. Flotation recovery is often optimal for particles of an intermediate size, with coarser particles exhibiting slow flotation kinetics, because of their size and poor liberation and fine particles exhibiting slow flotation kinetics because of poor flotation collision efficiency. Thus, the ore is preferably comminuted to a particle size P80 within the range of 1-150 µm, preferably 10-100 µm in step (a2). The optimization of the particle size distribution is within the routine capabilities of the skilled person. The expression "P80" as used herein in the context of particle size is the screen size through which 80% of the particles will pass.

As indicated herein elsewhere, froth flotation separation is often applied in the form of a multi-stage process. Hence, embodiments of the invention are also envisaged wherein the pulp provided in step (a) is a mineral concentrate obtained from the selective separation of minerals in a previous froth flotation stage. This previous froth flotation stage may be a froth flotation process according to the invention (employing the first depressant) or a froth flotation stage employing another depressant.

The pulp provided in step (a) typically has a solids content within the range of 1-70wt.%, such as 2-60 wt.%, preferably 5-35 wt.%. In preferred embodiments the first mineral is present in the pulp provided in step (a) in an amount within the range of 0.01-10 wt.% (based on dry weight), preferably 0.1-5 wt.% (based on dry weight). In case the process according to the invention is used in the context of mineral recovery from tailings the concentration of the first mineral may be lower, for example within the range of 0.001-2 wt.% (based on dry weight), preferably 0.005-0.5 wt.%.

The pulp provided in step (a) typically comprises a collector, for example a collector which was used in a previous froth flotation stage to separate gangue material from valuable minerals. Hence, in some embodiments the process of the invention is provided wherein the pulp provided in step (a) comprises a collector, preferably a collector selected from the group consisting of xanthates, xanthogen formates, thioureas, thionocarbamates, (di)thiophosphates, dithiophosphinates, N-alkoxycarbonyl dithiocarbamates, dialkyldithiocarbamates, mercaptobenzothioazoles, hydrocarbons (such as kerosene), nitriles and combinations thereof.

Suitable xanthate collectors include compounds of general formula R^{x}-O-(CS₂)-H, salts thereof or dimers thereof (also referred to as dixanthogens) wherein R^{x} is selected from the group consisting of C₁-C₁₂ alkyl, preferably R^{x} is selected from the group consisting of C₁-C₆ alkyl. The xanthate is preferably provided as an alkaline metal salt and/or an alkaline earth metal salt, more preferably a sodium, calcium, magnesium and/or potassium salt, most preferably a sodium and/or potassium salt. Exemplary xanthate collectors are Sodium isobutyl xanthate, Potassium amyl xanthate, Sodium isopropyl xanthate.

Suitable thionocarbamate collectors include dialkyl thionocarbamates, alkyl alkoxycarbonyl thionocarbamates, and alkyl allyl thionocarbamates having the general formula R^{y}-O-C(=S)-NHR^{Z} or salts thereof wherein R^{y} is a C₁-C₈ aliphatic hydrocarbyl, preferably a C₁-C₄ aliphatic saturated hydrocarbyl, and R^{Z} is selected from the group consisting of hydrogen, a C₁-C₈ aliphatic hydrocarbyl, a vinyl group, and a group of formula -COOR^{z'} wherein R^{z'} is a C₁-C₈ aliphatic hydrocarbyl, preferably R^{Z} is selected from the group consisting of hydrogen, a C₁-C₄ aliphatic saturated hydrocarbyl, a vinyl group, and a group of formula -COOR^{z'} wherein R^{z'} is a C₁-C₄ aliphatic hydrocarbyl. The expression an 'aliphatic saturated hydrocarbyl' is equivalent to an 'alkyl group'. In case R^{Z} is hydrogen, the thionocarbamate can be provided as a salt, preferably provided as preferably an alkaline metal salt and/or an alkaline earth metal salt, more preferably a sodium, calcium, magnesium and/or potassium salt, most preferably a sodium and/or potassium salt. An exemplary thionocarbamate collector is isopropyl ethyl thionocarbamate.

Suitable (di)thiophosphate collectors include compounds having the general formula R^{u}-O-PS₂-O-R^{v} and R^{u}-O-P(OR^{v})S-O-R^{w} or salts thereof wherein R^{u}, R^{v} and R^{w} are independently selected from the group consisting of hydrogen, and C₁-C₁₀ aliphatic or aromatic hydrocarbyls and at least one, preferably at least two of R^{u}, R^{v} and R^{w} are not hydrogen. Preferably R^{u}, R^{v} and R^{w} are independently selected from the group consisting of hydrogen and C₁-C₆ alkyl and at least one, preferably two of R^{u}, R^{v} and R^{w} are not hydrogen. The (di)thiophosphate can be provided as a salt, preferably provided as preferably an alkaline metal salt and/or an alkaline earth metal salt, more preferably a sodium, calcium, magnesium and/or potassium salt, most preferably a sodium and/or potassium salt. Exemplary (di)thiophospate collectors are sodium diisobutyl dithiophosphate abd disecondary butyl dithiophosphate (DBD).

Suitable dithiophosphinate collectors include compounds having the general formula R^{u}-PS₂-R^{v} or salts thereof wherein R^{u} and R^{v} are independently selected from the group consisting of hydrogen, and C₁-C₁₀ aliphatic or aromatic hydrocarbyls and at least one, preferably two of R^{u} and R^{v} are not hydrogen. Preferably R^{u} and R^{v} are independently selected from the group consisting of hydrogen and C₁-C₆ alkyl and at least one, preferably at least two of R^{u} and R^{v} are not hydrogen. The dithiophosphinate can be provided as a salt, preferably provided as preferably an alkaline metal salt and/or an alkaline earth metal salt, more preferably a sodium, calcium, magnesium and/or potassium salt, most preferably a sodium and/or potassium salt. An exemplary dithiophosphinate collector is sodium di(isobutyl)dithiophosphinate.

Suitable N-alkoxycarbonyl dithiocarbamates collectors include compounds of formula R^{m}O-C(=O)-NH-CS₂-Rⁿ are described in US8376142B2 and include compounds wherein R^{m} and Rⁿ are independently chosen from optionally substituted C₁-C₂₀ alkyl, optionally substituted C₆-C₂₀ aryl, optionally substituted C₂-C₂₀ alkenyl, and optionally substituted C₇-C₂₀ aralkyl, preferably R^{m} and Rⁿ are independently chosen from C₁-C₈ alkyl, phenyl, C₂-C₈ alkenyl and C₇-C₁₀ aralkyl. Exemplary dithiocarbamate N-alkoxycarbonyl dithiocarbamates are described in US8376142B2 column 3 lines 32-59.

Suitable dialkyldithiocarbamate collectors include compounds having the general formula R^{f}R^{g}N-CS₂H or salts thereof wherein R^{f} and R^{g} are independently chosen from C₁-C₁₂ alkyl, preferably C₁-C₆ alkyl. The dialkyldithiocarbamate can be provided as a salt, preferably provided as preferably an alkaline metal salt and/or an alkaline earth metal salt, more preferably a sodium, calcium, magnesium and/or potassium salt, most preferably a sodium and/or potassium salt. An exemplary dialkyldithiocarbamate collector is sodium dimethyl dithiocarbamate.

Suitable thiourea collectors include compounds having the general formula R^{f}R^{g}N-CS-NR^{h}Rⁱ wherein R^{f}, R^{g}, R^{h}, and Rⁱ are independently chosen from C₁-C₁₂ alkyl and C₁-C₁₂ alkylethoxycarbonyl, preferably C₁-C₆ alkyl and C₁-C₆ alkylethoxycarbonyl. An exemplary thiourea collector is butyl ethoxycarbonyl thiourea (BECTU).

Suitable xanthogen formate collectors include compounds of general formula R^{x}-O-(CS₂)-C(O)OR^{x'} or salts thereof wherein R^{x} is selected from the group consisting of C₁-C₁₂ alkyl, preferably R^{x} is selected from the group consisting of C₁-C₆ alkyl and R^{x'} is selected from the group consisting of hydrogen and C₁-C₁₂ alkyl, preferably R^{x'} is selected from the group consisting of hdyrogen and C₁-C₆ alkyl.

Suitable mercaptobenzothiazole collectors include 2-mercaptobenzothiazole and C₁-C₁₄ hydrocarbyl derivatives thereof, preferably C₁-C₆alkyl derivatives thereof.

Suitable nitrile collectors include long chain hydrocarbons having from 10 to 50 carbon atoms and comprising one, two or more nitrile functional groups, wherein optionally one or more carbon atoms in the hydrocarbon backbone is substituted by a nitrogen atom. Exemplary nitrile collectors are known under the tradenames Tecflote S10 and Tecflote S11 available from the company Nouryon.

As will be understood by the skilled person in light of the present disclosure, many process variations are possible without deviating from the spirit and scope of the present invention, and it is impossible to list all of these. For example, the pulp provided in step (a) may have been further pretreated by acid digestion, attrition scrubbing, heat treatment, and combinations thereof. Furthermore, the pulp provided in step (a) typically comprises, aside from the ingredients explicitly recited herein elsewhere, 0-10 wt.% (by total weight of the pulp) other additives such as kerosene, diesel oil, organic solvents, polymers, remnants of explosives, pH modifiers, ORP modifiers, etc.

The first depressant may be added to the pulp before and/or during the froth flotation of step (c). As will be understood by the skilled person, many possible addition schemes exist for the first depressant. For example, it is possible to add a portion of first depressant before the froth flotation of step (c), and add the remainder of the first depressant during the froth flotation of step (c). Alternatively it is possible to add the complete amount of first depressant employed in the process of the present invention before the froth flotation of step (c), whereby the addition may take place gradually over a prolonged period of time, or it may take place as a single addition. The first depressant may be added as a pure compound, as a solution (aqueous or otherwise) or as a blend with other compounds. The invention is not particularly limited with regard to the method of addition of the first depressant.

In accordance with preferred embodiments of the invention, the total amount of first depressant added to the pulp is within the range of 0.1-100 Ibs/ton (based on dry weight of the pulp), preferably 0.5-50 Ibs/ton (based on dry weight of the pulp). The expression "ton" refers to a US ton and equals 2000 lbs. Expressed in ppm this means that in accordance with preferred embodiments of the invention the total amount of first depressant added to the pulp is within the range of 50-50000 ppm (w/w, based on dry weight of the pulp), preferably 250-25000 ppm (w/w, based on dry weight of the pulp).

As is shown in the appended examples, the present inventors have found that the depressants of formula (I) exhibit particular and surprising properties which are desirable in the context of sulfide mineral recovery. For example, several compounds have been shown to achieve >80% sulfide mineral (chalcopyrite) depression while allowing another sulfide mineral (molybdenite) to be recovered in the froth. Hence, in accordance with preferred embodiments of the invention, the first mineral is a sulfide mineral, preferably a molybdenum sulfide mineral. A complete list of sulfide minerals can be found in mindat.org. Preferably the first mineral is a sulfide mineral selected from the group consisting of Acanthite, Chalcocite, Bornite, Galena, Sphalerite, Chalcopyrite, Pyrrhotite, Millerite, Pentlandite, Covellite, Cinnabar, Realgar, Orpiment, Stibnite, Pyrite, Marcasite, Molybdenite, Cobaltite, Arsenopyrite, Gersdorffite, Pyrargyrite, Proustite, Tetrahedrite, Tennantite, Enargite, Bournonite, Jamesonite, Cylindrite, digenite, geerite, Mackinawite, reigite, spionkopite, Troilite, villamaninite, yarrowite, and combinations thereof. In accordance with highly preferred embodiments of the present invention, the first mineral is molybdenite.

In some embodiments, at least part of the first mineral is formed in-situ, for example by chemical conversion of one mineral into another during froth flotation. For example, in case the first mineral is a sulfide mineral, in-situ formation of the first mineral is possible when a sulfidizing agent (such as a polysulfide) is added to the pulp in order to convert any oxidized or hydrolyzed sulfide minerals back into their corresponding sulfides.

The pulp provided in step (a) typically has a pH greater than 7, preferably greater than 8. In preferred embodiments the process is performed such that the pH of the pulp during step (c) is maintained within the range of 8-13, preferably within the range of 8.5-11. The pH adjustment may take place before, during or after step (b). Suitable pH adjusting agents are for example Ca(OH)₂, sulfuric acid, CO₂, NaOH and/or KOH.

It will be understood by the skilled person that in order to achieve a meaningful recovery of the first mineral, the froth flotation of step (c) is performed such that the first mineral accumulates in the froth, thereby achieving a higher concentration of the first mineral in the froth than in the bulk pulp. Hence, step (d) of the method according to the invention, recovering the froth, will provide a second pulp comprising the first mineral which has an increased concentration of the first mineral (based on dry weight) compared to the pulp provided in step (a). For the sake of clarity it is noted that the froth, once it is recovered and no longer being aerated, will settle into a pulp. In embodiments the process is performed such that the concentration of the first mineral in the second pulp obtained from recovering the froth in step (d) is within the range of 2-40 wt.% (by total weight of pulp), preferably within the range of 5-25 wt.%. In embodiments the process is performed such that the recovery of the first mineral is more than 40%, preferably more than 60%, more preferably more than 85%. In highly preferred embodiments the process is performed such that the ratio of the concentration (based on dry weight) of the first mineral in the second pulp obtained from recovering the froth in step (d) to the concentration (based on dry weight) of the first mineral in the pulp provided in step (a), is more than 1.5, preferably more than 2, more preferably more than 4, most preferably more than 5.

Since achieving a perfectly complete mineral separation by froth flotation is impossible, as indicated herein elsewhere, froth flotation separation is often applied in the form of a multi-stage process. Hence, according to preferred embodiments of the invention the second pulp obtained from recovering the froth in step (d) can be provided as the pulp for a next froth-flotation stage, preferably a next froth-flotation stage according to the invention.

In accordance with highly preferred embodiments of the invention, the solids comprising the first mineral further comprises a second mineral, different from the first mineral. The compounds of formula (I) were found to be highly efficient selective depressants. Hence, the process typically comprises depressing the second mineral into the pulp. According to preferred embodiments, the solids comprising the first mineral further comprises a second mineral and the process comprises depressing the second mineral into the pulp, wherein the second mineral is a sulfide mineral, preferably a copper and/or iron sulfide mineral, which is different from the first mineral. In embodiments the second mineral is a sulfide mineral selected from the group consisting of Acanthite, Chalcocite, Bornite, Galena, Sphalerite, Chalcopyrite, Pyrrhotite, Millerite, Pentlandite, Covellite, Cinnabar, Realgar, Orpiment, Stibnite, Pyrite, Marcasite, Molybdenite, Cobaltite, Arsenopyrite, Gersdorffite, Pyrargyrite, Proustite, Tetrahedrite, Tennantite, Enargite, Bournonite, Jamesonite, Cylindrite, digenite, geerite, Mackinawite, reigite, spionkopite, Troilite, villamaninite, yarrowite, and combinations thereof, preferably selected from the group consisting of chalcocite, villamaninite, covellite, yarrowite, spionkopite, geerite, anilite, digenite, reigite, Pyrrhotite, Troilite, Mackinawite, Marcasite, Pyrite and combinations thereof, and the second mineral is different from the first mineral.

Examples of suitable combinations of first mineral and second mineral in the process of the present invention are as follows:

| **First mineral** | **Second mineral** |
|---|---|
| Molybdenite | Chalcopyrite |
| Molybdenite | Chalcocite |
| Molybdenite | Covellite |
| Molybdenite | Pyrite |
| Molybdenite | Bornite |
| Molybdenite | Sphalerite |

In accordance with highly preferred embodiments of the present invention, the solids comprising the first mineral further comprises a second mineral and the process comprises depressing the second mineral into the pulp, wherein the first mineral is molybdenite and the second mineral is chalcopyrite. Accordingly, it is preferred that the pulp provided in step (a) is a Cu/Mo concentrate obtained by separation of gangue material from Cu/Mo ore, preferably from an ore comprising molybdenite and chalcopyrite. Such a Cu/Mo concentrate typically has a Cu concentration within the range of 15-40 wt.% (based on dry weight), preferably 25-35 wt.% (based on dry weight) and a Mo concentration within the range of 0.01-10 wt.% (based on dry weight), preferably 0.1-5 wt.% (based on dry weight).

Embodiments of the invention are also envisaged wherein the pulp provided in step (a) is a mineral concentrate obtained from the selective separation of the first and second minerals in a previous froth flotation stage, wherein the previous froth flotation stage also entailed depressing the second mineral. This previous froth flotation stage may be a froth flotation process according to the invention (employing the first depressant) or a froth flotation stage employing another depressant. For example, after gangue separation, a first froth flotation separation stage may be applied employing NaHS as depressant, wherein the second mineral is depressed and the froth comprising the first mineral is collected to obtain a pulp comprising the first mineral, which is provided as the pulp in step (a) of the process of the present invention.

The froth may be produced employing any suitable gas, such as air, oxygen gas, nitrogen gas, CO₂ gas or combinations thereof. Preferably the froth is produced employing air, nitrogen gas, CO₂ gas of combinations thereof, more preferably air.

The pulp provided in step (a) may comprise a frothing agent and/or the process of the invention may comprise a step of adding a frothing agent. Suitable frothing agents are known to the skilled person, for example
- straight or branched chain C₃-C₈ alcohols, such as C₆₋₈ alkanols, 2-ethyl hexanol and 4-methyl-2-pentanol (MIBC);
- alkylphenols, such as cresol, xylenol;
- terpenic alcohols, such as alpha-terpineol;
- terpenic hydrocarbons;
- cresylic acids;
- alkyl sulfonates, such as C₁-C₈ alkyl sulfonates;
- hydroxy ketones, such as beta-hydroxy ketones having C₁-C₈ hydrocarbon substitutents;
- monoglycols, such as monoethylene glycol, 1,3-butanediol, propane-1,2-diol; and
- polyglycols, such as diethylene glycol or ethyleneoxide-propylene oxide oligomers.

The pulp provided in step (a) may comprise a second depressant and/or the process of the invention may comprise a step of adding a second depressant. Suitable depressants are known to the skilled person, and include but are not limited to:
- trithiocarbonates (such as those described in US4,533,466, in particular disodium carboxymethyl trithiocarbonate),
- NaHS,
- Nokes reagent,
- ferrocyanides,
- polysulfides,
- bisulfites,
- polymers comprising an allyl thiourea functional group and a hydrophilic acrylamide group (such as the polymers described in US4,888,106 and US4,966,938 and commercialized as AERO^{®} 7260 HFP by Cytec Industries Inc., Woodland Park, NJ),
- nonpolar oils (such as diesel, kerosene),
and combinations thereof. The ferrocyanides, polysulfides and bisulfites are typically added in the form of a salt, such as an alkaline metal or an alkaline earth metal salt. Ammonium salts of polysulfides have inherent HSE issues similar to NaHS, and in some environments are more severe for ammonium polysulfide than NaHS due to the higher vapor pressures of H₂S and NH₃ as well as the subsequent higher H₂S evolution rate when pH is decreased. In preferred embodiments substantially no ammonium polysulfide is present.

In particular embodiments, the process of the invention is provided further comprising the step of:
(b2) adding a second depressant to the pulp provided in step (a);
with the provisio that when R¹ comprises no other functional groups than alcohols, the process does not comprise the use of a second depressant selected from polymers comprising an allyl thiourea functional group and a hydrophilic acrylamide group;
wherein step (b2) may be performed before, during and/or after step (b). Preferably, the second depressant is selected from the group consisting of NaHS, Nokes reagent, ferrocyanides, polysulfides, bisulfites, trithiocarbonates, polymers comprising an allyl thiourea functional group and a hydrophilic acrylamide group, nonpolar oils and combinations thereof. The in-situ formation of a second depressant by addition of a non-depressant reagent to the pulp which is converted into, or effects formation of, a second depressant in the pulp is also construed as the addition of a second depressant.

As is shown in the appended examples, the present inventors have found that both alone and in combination with NaHS, the first depressant can achieve excellent performance, such that it can be considered a partial or complete NaHS replacement. Hence, in some embodiments the second depressant is selected from polysulfides, bisulfites, NaHS and combinations thereof, preferably the second depressant is NaHS. This embodiment has the advantage that existing installations do not need to be completely converted to the depressants of the present invention, which may be more economical, while still allowing a significantly reduced NaHS consumption, reducing the negative HSE impact of NaHS. In other embodiments the second depressant is substantially free of, preferably completely free of, NaHS. In some embodiments the complete froth flotation process of the invention does not utilize NaHS. These embodiments have the advantage that the negative HSE impact of NaHS can be completely mitigated.

In some preferred embodiments, the process of the invention is provided further comprising the step of:
(b2) adding NaHS to the pulp provided in step (a);
wherein step (b2) may be performed before, during and/or after step (b), and wherein the first depressant is as described herein before.

In some embodiments of the present invention the pulp provided in step (a) may be free of depressants other than the first depressant and/or the process of the invention does not comprise the use of any other depressant than the first depressant.

### The compositions of the invention

The invention also concerns compositions relating to the use of the dithiocarbamic acids or salts thereof of formula (I) in the field of mining as detailed in the claims. In another aspect of the invention there is thus provided a composition comprising a first depressant and further comprising one, two, or three of:
- a collector as described herein
- a second depressant as described herein; and
- a mineral, preferably the first mineral as described herein;

wherein the first depressant consists of one or more dithiocarbamic acids or salts thereof of formula (I) as described in claim 1 of the appended set of claims,
wherein
   R¹ represents a first substituent having from 2 to 5 carbon atoms and comprising one or two alcohols; or
   R¹ and R² are identical; or
   R¹ and R² are connected to form a heterocyclic 5 or 6 membered ring comprising 2 heteroatoms selected from N and O, which is optionally substituted with a single functional group selected from -C₁-C₄ alkyl, preferably the heterocyclic ring is substituted with a single methyl group; or
   the dithiocarbamic acids or salts thereof of formula (I) are selected from the group consisting of compounds 1, 3-18, 20 acid as defined herein elsewhere,
with the provisio that the first depressant is not a compound of formula (I) wherein R¹ comprises no other functional groups than alcohol.

In preferred embodiments of the invention, the composition comprising the first depressant is provided which is a mineral pulp, comprising a sulfide mineral, preferably the first mineral as described herein, in an amount of at least 0.01 wt.% (based on total weight of the mineral pulp), preferably at least 0.1 wt.% and optionally further comprising
- a collector as described herein in an amount of at least 10 ppm (w/w, based on total weight of mineral pulp); and/or
- a second depressant as described herein in an amount of at least 10 ppm (based on total weight of the mineral pulp);
wherein the first depressant is preferably present in an amount of at least 10 ppm (based on total weight of the mineral pulp).

In preferred embodiments of the invention, the composition comprising the first depressant is provided which is a mining additive composition comprising a second depressant as described herein in an amount of at least 5 wt.% (based on total weight of the additive composition excluding solvents), preferably at least 10 wt.% (based on total weight of the additive composition excluding solvents); wherein the first depressant is preferably present in an amount of at least 5 wt.% (based on total weight of the additive composition excluding solvents), preferably at least 20 wt.% (based on total weight of the additive composition excluding solvents). The mining additive composition is preferably substantially free of other additives. For example, the combined amount of depressants is preferably more than 90 wt.% (by total weight of the additive composition excluding solvents), preferably more than 95 wt%, more preferably more than 99 wt.%. In some embodiments the mining additive composition essentially consists of (i) the first depressant, (ii) the second depressant, and (iii) solvent, wherein the solvent is preferably selected from water, alcohols, hydrocarbons and combinations thereof, preferably water or alcohol. Examples of suitable alcohol solvents are C₁-C₆ monoalcohols, and monoglycols, such as monoethylene glycol, 1,3-butanediol and propane-1,2-diol. The mining additive composition may be provided in the form of a concentrate, wherein the first depressant is present in an amount of more than 50 wt.% (by total weight of the concentrate) and wherein the total amount of solvent is less than 40 wt.% (by total weight of the concentrate).
In particular embodiments the compositions described herein are provided with the provisio that the first depressant is not *N*-(2-Aminoethyl)carbamodithioic acid or a salt thereof or a compound of formula (I) wherein R¹ comprises no other functional groups than alcohols.

### The use of the dithiocarbamic acids or salts thereof of formula (I) of the invention

In another aspect the invention concerns the use of a dithiocarbamic acid or salt thereof of formula (I) as described in any one of claims 1-8 of the appended set of claims in the recovery of minerals with the provisio that the dithiocarbamic acid or salt thereof is not *N*-(2-Aminoethyl)carbamodithioic acid or a salt thereof or a compound of formula (I) wherein R¹ comprises no other functional groups than alcohols. Preferably the use is in the froth flotation recovery of minerals, preferably of sulfide minerals. More preferably, the use is as a depressant of a second mineral as described herein in the froth flotation recovery of a first mineral as described herein.

In particular embodiments the invention concerns the use of a dithiocarbamic acid or salt thereof of formula (I) as described herein as a depressant of a copper and/or iron sulfide mineral, preferably of chalcopyrite in the froth flotation recovery of another sulfide mineral, preferably of molybdenite.

In particular embodiments the uses described herein are provided with the provisio that the dithiocarbamic acid or salt thereof is not *N*-(2-Aminoethyl)carbamodithioic acid or a salt thereof or a compound of formula (I) wherein R¹ comprises no other functional groups than alcohols.

### The kit of parts according to the disclosure

An aspect the disclosure, but not the claims, concerns a kit of parts comprising a composition (A) comprising the first depressant as described herein and instructions for use of the composition (A) as a depressant in froth flotation recovery of minerals.

Another aspect of the disclosure, but not the claims, concerns a kit of parts comprising
- a composition (A) comprising the first depressant as described herein;
- a composition (B) comprising a second depressant, preferably a second depressant as described herein; and
- optionally instructions for use of the composition (A) as a depressant in froth flotation recovery of minerals.

Preferably, the concentration of the first depressant in composition (A) is more than 10 wt.% (by total weight of composition (A), preferably more than 50 wt.%.

Preferably, the concentration of the second depressant in composition (B) is selected from the gorup consisting of NaHS, Nokes reagent, ferrocyanides, polysulfides, bisulfites, trithiocarbonates and combinations thereof, preferably NaHS.

Preferably the instructions are for use of the composition (A) in the froth flotation recovery of minerals, preferably of sulfide minerals. More preferably, the instructions are for use of the composition (A) as a depressant of a second mineral as described herein in the froth flotation recovery of a first mineral as described herein. In particular embodiments of the disclosure, but not the claims, the instructions are for use of the composition (A) as a depressant of a copper and/or iron sulfide mineral, preferably of chalcopyrite in the froth flotation recovery of another sulfide mineral, preferably of molybdenite.

In particular embodiments the kit of parts described herein are provided with the provisio that the first depressant is not *N*-(2-Aminoethyl)carbamodithioic acid or a salt thereof or a compound of formula (I) wherein R¹ comprises no other functional groups than alcohols.

### Dithiocarbamates of formula (I) as such

The present inventors believe that some of the dithiocarbamic acids or salts thereof of formula (I) found in the course of this work have not been described before. In another aspect the invention thus concerns a dithiocarbamic acid or salt thereof of formula (I) as described herein before, in particular 3-amino-1,2-propanediol dithiocarbamic acid or a salt thereof, 2-amino-2-methyl-1,3,-propanediol dithiocarbamic acid or a salt thereof, or N-phenylethylenediamine dithiocarbamic acid or a salt thereof, preferably 3-amino-1,2-propanediol dithiocarbamic acid or a salt thereof. These compounds are also referred to herein as compound 3 acid or a salt thereof, compound 7 acid or a salt thereof, or compound 11 acid or a salt thereof. These compounds are also referred to herein by their systematic name as 2,3-dihydroxypropylcarbamodithioic acid or a salt thereof, 2-amino-2-methyl-1,3,-propanediol carbamodithioic acid or a salt thereof, or phenyl(2-aminoethyl)carbamodithioic acid or a salt thereof. As is shown in the below table, all these names concern the same three compounds identified by their structural formula. The present invention claims any one of these three compounds as such, as well as any use thereof.

| **Compound name** | **Compound Structure** | **CAS** | **Systematic name** |
|---|---|---|---|
| 3-amino-1,2-propanediol dithiocarbamic acid (**compound 3 acid**) | 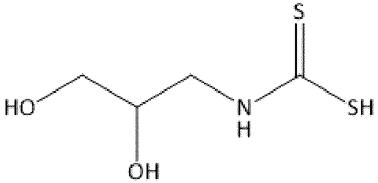 | NA | 2,3-dihydroxypropylcarb amodithioic acid |
| 2-amino-2-methyl-1,3,-propanediol dithiocarbamic acid (**compound 7 acid**) | 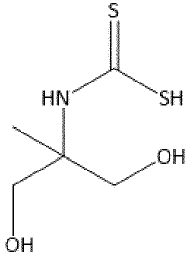 | NA | 2-amino-2-methyl-1,3,-propanediol carbamodithioic acid |
| N-phenylethylenedia mine dithiocarbamic acid (**compound 11 acid**) | 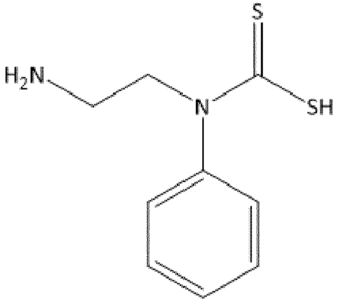 | NA | phenyl(2-aminoethyl)carbamo dithioic acid |

### Methods of synthesis of dithiocarbamic acids or salts thereof of formula (I)

Another aspect of the disclosure, but not the claims, provides a method of synthesizing a dithiocarbamic acid or salt thereof of formula (I) as described herein, comprising the steps of:
(i) providing an amine of formula R¹R²NH;
(ii) providing CS₂; and
(iii) reacting the amine of step (i) with the CS₂ of step (ii) under suitable conditions to form the dithiocarbamic acid or salt thereof of formula (I);
wherein optionally a base, such as KOH or NaOH, preferably NaOH is added
- to the amine of step (i) before step (iii);
- to the CS₂ of step (ii) before step (iii); and/or
- to the reaction product of step (iii).

Preferably the base is added to the reaction product of step (iii). Preferably the reaction is performed employing a solvent consisting of ethanol and/or water, preferably employing a solvent consisting of water.

### EXAMPLES

### Example 1: synthesis of N-substituted dithiocarbamates:

The dithiocarbamic acids or salts thereof used in the various examples were synthesized according to a known reaction scheme wherein the R¹R²NH is reacted with CS₂, R¹ and R² being as described herein before.

To a round-bottom flask fitted with a stirrer, condenser, and thermometer, amine is first placed in solvent. The reaction flask is chilled in an ice bath during the dropwise addition of equimolar carbon disulfide (CS₂) so that the reaction temperature does not exceed 38°C. The reaction is then allowed to stir heated (between 32-38°C) for a minimum of one hour or until the solution has visibly reacted and the CS₂ is no longer beaded in the flask. After the reaction is complete, an equimolar amount of base is added dropwise. The solution is again heated between 32-38°C for one hour and then purged with nitrogen. All syntheses use the same ratio of starting materials, 0.1 mol of the amine, 0.1 mol CS2, 0.1 mol of 50% NaOH in solvent. The final reaction product is a 30-60% salt solution. In some instances a solid is formed which is then isolated. Formation of the dithiocarbamic acid or salt thereof can be confirmed via two distinctive absorption peaks in the UV-Vis region around ^{~}250nm and ^{~}280nm. Masses of starting materials and final product concentrations are listed in Table 1. It is noted that for some syntheses the order of addition of the different compounds was reversed, first adding amine and base, followed by the addition of carbon disulfide. It is within the routine capabilities of the skilled person to synthesize other compounds of formula (I) based on the guidance provided herein.

**Table 1. Starting reactants and final concentrations of N-substituted dithiocarbamate salts.**

| N-substituted dithiocarbamate salt | carbon disulfide (g) | amine (g) | 50% sodium hydroxide (g) | Solvent | Final Concentration (%wt) | Density (g/mL) |
|---|---|---|---|---|---|---|
| sodium diethanolamine dithiocarbamate (**compound 1**) | 7.61 | 10.51 | 8.00 | water | 0.40 | 1.265 |
| sodium 3-amino-1,2- propanediol dithiocarbamate (**compound 3**) | 7.61 | 9.11 | 8.00 | water | 0.40 | 1.171 |
| sodium 2-(methylamino)ethanol dithiocarbamate (**compound 4**) | 7.61 | 7.51 | 8.00 | water | 0.63 | 1.241 |
| sodium bis(2-hydroxypropyl)amine dithiocarbamate (**compound 5**) | 7.61 | 13.32 | 8.00 | water | 0.47 | 1.124 |
| sodium bis(2-methoxyethyl)amine dithiocarbamate (**compound 6**) | 7.61 | 13.32 | 8.00 | water | 0.47 | 1.123 |
| sodium morpholine dithiocarbamate (**compound 8**) | 7.61 | 8.71 | 8.00 | ethanol | 100 | - |
| sodium n-methylpiperazine dithiocarbamate (**compound 9**) | 7.61 | 10.02 | 8.00 | ethanol | 100 | - |
| sodium (2-methoxyethyl)methylamine dithiocarbamate (**compound 12**) | 7.61 | 8.91 | 8.00 | water | 0.42 | 1.138 |
| sodium 3,3'-iminobis(n,n-dimethylpropylamine) dithiocarbamate (**compound 13**) | 7.61 | 18.73 | 8.00 | ethanol | 0.52 | 1.020 |
| sodium 2-(ethylamino)ethanol dithiocarbamate (**compound 14**) | 7.61 | 8.91 | 8.00 | water | 0.42 | 1.048 |
| sodium ethanolamine dithiocarbamate (**compound 16**) | 7.61 | 6.11 | 8.00 | water | 0.40 | 1.280 |
| sodium piperazine dithiocarbamate (**compound 18**) | 7.61 | 8.61 | 8.00 | water | 100 | - |

### Example 2: Hallimond Tube - depression of chalcopyrite

Approximately 2.5 g of 95% pure chalcopyrite (CuFeS₂, size fraction -105+45 µm, deslimed) is placed in a beaker with 50mL of 0.001 M KNO₃ solution and stirred for two minutes. KNO₃ is added to increase the ionic strength of the solution to more closely resemble real-life mining operations. Afterward, a stock solution of collector is added to the slurry for a final concentration of 0.05 mmol/L along with 1.7µL of methyl isobutyl carbinol (MIBC, a frother) and the mixture is allowed to condition for another five min. Frother and collector are added in order to closely resemble real-life mining operations. Next, a sample of stock solution of dithiocarbamate salt is added to the mixture and the slurry is conditioned for 10 min. The slurry is then transferred to a Hallimond tube along with an additional 70mL of 0.001 M KNO₃ solution and floated for three minutes. The concentrate is obtained from the collection arm. Both concentrate and tail are filtered and weighed.

Table 2 shows the results obtained with potassium amyl xanthate (PAX) as collector.

Table 3 shows the results obtained with potassium amyl xanthate (PAX) as collector for various dithiocarbamate depressant concentrations.

Table 4 shows the results obtained with Isopropyl ethyl thionocarbamate as collector.

It can be observed in Tables 2 and 3 that various compounds according to the present invention, such as sodium 3-amino-1,2- propanediol dithiocarbamate (**compound 3**), sodium morpholine dithiocarbamate (**compound 8**), and sodium n-methylpiperazine dithiocarbamate (**compound 9**) show excellent performance in depressing chalcopyrite both when used as the sole depressant, or when used in combination with sodium hydrosulfide (NaSH). In particular, the compounds consistently outperform sodium hydrosulfide (NaSH) and significantly outperform sodium hydrosulfide (NaSH) when employed at low concentrations. It can be observed in Table 4 that also with other collectors, such as ethyl thionocarbamate, the compounds of the present invention have excellent performance in depressing chalcopyrite.

**Table 2. Chalcopyrite Recovery for different depressants with Potassium Amyl Xanthate as a Collector**

| Test No. | Depressant | Dosage (mmol/L) | % Mass Recovery Chalcopyrite | | | | | | |
|---|---|---|---|---|---|---|---|---|---|
| | | | Run 1 | Run 2 | Run 3 | Run 4 | Run 5 | Avg. | Std. Dev. |
| Control | | | | | | | | | |
| 1 | No depressant | - | 77.52 | 60.01 | 84.64 | 69.10 | 86.99 | 79.38 (9 runs) | 9.41 |
| 1 | No depressant | - | 80.16 | 76.82 | 87.05 | 92.09 | | | |
| 2 | sodium hydrosulfide (NaSH) | 0.05 | 27.23 | 75.09 | 82.87 | 35.17 | 91.00 | 62.27 | 25.99 |
| 3 | sodium hydrosulfide (NaSH) | 0.20 | 13.83 | 12.17 | 5.40 | 14.34 | - | 11.44 | 3.57 |

| Invention | | | | | | | | | |
|---|---|---|---|---|---|---|---|---|---|
| 4 | sodium 3-amino-1,2-propanediol dithiocarbamate (**compound 3**) | 0.10 | 7.39 | 4.08 | - | - | - | 5.73 | 1.65 |
| 5 | sodium morpholine dithiocarbamate (**compound 8**) | 0.10 | 4.12 | 2.81 | - | - | - | 3.46 | 0.65 |
| 6 | sodium n-methylpiperazine dithiocarbamate (**compound 9**) | 0.10 | 10.88 | 28.92 | - | - | - | 19.90 | 9.02 |
| 7 | sodium 3-amino-1,2-propanediol dithiocarbamate (**compound 3**) and sodium hydrosulfide (**NaSH**) | 0.10 / 0.05 | 8.03 | 6.50 | - | - | - | 7.26 | 0.76 |
| 8 | sodium morpholine dithiocarbamate (**compound 8**) and sodium hydrosulfide (**NaSH**) | 0.10 / 0.05 | 5.47 | 19.96 | - | - | - | 12.71 | 7.24 |
| 9 | sodium n-methylpiperazine dithiocarbamate (**compound 9**) and sodium hydrosulfide (**NaSH**) | 0.10 / 0.05 | 13.33 | 20.09 | - | - | - | 16.71 | 3.38 |

**Table 3. Effect of Concentration on depressant performance with Potassium Amyl Xanthate as a Collector**

| | | | % Mass Recovery Chalcopyrite | | | |
|---|---|---|---|---|---|---|
| Test No. | Depressant | Dosage (mmol/L) | Run 1 | Run 2 | Avg. | Std. Dev. |
| | Control | | | | | |
| 10 | - | - | 87.04 | 83.36 | 85.20 | 1.84 |
| 11 | sodium hydrosulfide (NaSH) | 0.05 | 81.06 | 40.93 | 60.99 | 20.07 |
| 12 | sodium hydrosulfide (NaSH) | 0.15 | 27.93 | 13.69 | 20.81 | 7.12 |
| 13 | sodium hydrosulfide (NaSH) | 0.20 | 16.49 | 10.75 | 13.62 | 2.87 |

| | Invention | | | | | |
|---|---|---|---|---|---|---|
| 14 | sodium 3-amino-1,2-propanediol dithiocarbamate (**compound 3**) | 0.05 | 24.97 | 4.92 | 14.95 | 10.02 |
| 15 | | 0.10 | 7.39 | 4.08 | 5.73 | 1.65 |
| 16 | | 0.15 | 8.05 | 3.75 | 5.90 | 2.15 |
| 17 | | 0.20 | 8.80 | 3.83 | 6.32 | 2.48 |
| 18 | sodium morpholine dithiocarbamate (**compound 8**) | 0.05 | 17.91 | 11.98 | 14.94 | 2.96 |
| 19 | | 0.10 | 4.12 | 2.81 | 3.46 | 0.65 |
| 20 | | 0.15 | 14.00 | 6.13 | 10.07 | 3.93 |
| 21 | | 0.20 | 8.65 | 4.00 | 6.32 | 2.32 |

**Table 4. Chalcopyrite Recovery for different depressants with Isopropyl Ethyl Thionocarbamate as a Collector**

| | | | % Mass Recovery Chalcopyrite |
|---|---|---|---|
| Test No. | Depressant | Dosage (mmol/L) | |
| | Control | | |
| 26 | - | - | 89.48 |
| 27 | sodium hydrosulfide (NaSH) | 0.05 | 60.38 |
| 28 | sodium hydrosulfide (NaSH) | 0.20 | 22.93 |

| | Invention | | |
|---|---|---|---|
| 29 | sodium 3-amino-1,2- propanediol dithiocarbamate (**compound 3**) | 0.20 | 20.45 |
| 30 | sodium morpholine dithiocarbamate (**compound 8**) | 0.20 | 9.45 |
| 31 | sodium n-methylpiperazine dithiocarbamate (**compound 9**) | 0.20 | 30.84 |

### Example 3: Hallimond Tube - flotation of molybdenite

For molybdenite flotation using a Hallimond Tube, the procedure is nearly identical to the procedure described in example 3. However, no additional desliming is conducted before flotation.

Approximately 2.5 g of 95% pure molybdenite (size fraction -105+45 µm) is placed in a beaker with 50mL of 0.001 M KNO₃ solution and stirred for two minutes. KNO₃ is added to increase the ionic strength of the solution to more closely resemble real-life mining conditions. Afterward, a stock solution of kerosene emulsion (collector) is added to the slurry for a final concentration of 20 ppm along with 1.7µL of methyl isobutyl carbinol (MIBC, a frother) and the mixture is allowed to condition for another five min. Frother and collector are added in order to closely resemble real-life mining operations. Next, a sample of stock solution of dithiocarbamate salt is added to the mixture and the slurry is conditioned for 10 min. The slurry is then transferred to a Hallimond tube along with an additional 70mL of 0.001 M KNO₃ solution and floated for three minutes. The concentrate is obtained from the collection arm. Both concentrate and tail are filtered and weighed.

Table 5 shows the results obtained. It can be observed from Table 5 that next to the excellent chalcopyrite depression shown in Example 2, the depressants according to the present invention also exhibit excellent molybdenite recovery. This effectively shows highly efficient selective depression of different sulfide minerals can be achieved with the depressants according to the present invention.

**Table 5. Effect of Concentration of N-Substituted Dithiocarbamate Salts on Molybdenite Recovery Using Kerosene as a Collector**

| | | | % Mass Recovery Molybdenite |
|---|---|---|---|
| Test No. | Depressant | Dosage (mmol/L) | |
| | Control | | |
| 32 | - | - | 62.41 |
| 33 | sodium hydrosulfide (NaSH) | 0.20 | 76.28 |

| | Invention | | |
|---|---|---|---|
| 34 | sodium 3-amino-123- propanediol dithiocarbamate (**compound 3**) | 0.10 | 74.91 |
| 35 | sodium morpholine dithiocarbamate (**compound 8**) | 0.10 | 63.26 |
| 36 | sodium n-methylpiperazine dithiocarbamate (**compound 9**) | 0.10 | 44.48 |

### Example 4: Separation of copper sulfide and molybdenum sulfide in a Denver flotation cell

A slurry (approx. 500mL of ~62% solids) containing mineral concentrate is placed into a 1.2L Denver flotation cell. The concentrate was obtained from a copper mine and is a copper sulfide and molybdenum sulfide containing concentrate obtained after gangue separation from the original ore. The majority of the copper mineral contained in the concentrate is chalcopyrite, and the gangue content was low in view of a previous gangue separation step using a xanthate collector. The remaining volume is filled with water to within 1-2 inches of the top of the cell. Depressant reagent is added to the slurry and conditioned (900 rpm stirring) for 4 minutes, then floated for 10 minutes (nitrogen, 350mL/min). Two concentrates are collected at 5 and 10 minutes, respectively. The pH was monitored throughout each experiment. The new concentrate and remaining tailings are filtered, weighed, and analyzed.

Table 6 shows the results obtained. It can be observed from table 6 that the depressants according to the present invention exhibit excellent performance in real-world conditions, and can also be used for significantly reducing the amount of sodium hydrosulfide (NaSH) required for achieving good Cu-Mo sulfide separation.

**Table 6. Effect of depressants on Copper/Molybdenum separation**

| | | | | % Recovery Concentrate | |
|---|---|---|---|---|---|
| Test No. | Depressant | Dosage* (lbs/ton) | Dosage* (ppm w/w) | Cu | Mo |
| | Control | | | | |
| 37 | - | - | | 88.27 | 78.11 |
| 38 | sodium hydrosulfide (NaSH) | 0.9 | 450 | 83.58 | 80.26 |
| 39 | sodium hydrosulfide (NaSH) | 1.8 | 900 | 12.09 | 95.33 |

| | Invention | | | | |
|---|---|---|---|---|---|
| 40 | sodium diethanolamine dithiocarbamate (**compound 1**) and sodium hydrosulfide (NaSH) | 0.9/0.9 | 450/450 | 13.49 | 90.83 |
| 41 | sodium diethanolamine dithiocarbamate (**compound 1**) and sodium hydrosulfide (NaSH) | 0.9/1.35 | 450/675 | 10.66 | 92.87 |

| | | | | | |
|---|---|---|---|---|---|
| *dosage based on dry weight. Ton = US ton (2000 lbs). | | | | | |

## Claims

1. A process for recovering a first mineral comprising the steps of:
(a) providing a pulp comprising solids and water, wherein the solids comprise the first mineral;
(b) adding a first depressant to the pulp;
(c) subjecting the pulp to a froth flotation process to produce a froth comprising the first mineral; and
(d) recovering the froth;
wherein the first mineral is a sulfide mineral, and the first depressant consists of one or more dithiocarbamic acids or salts thereof of formula (I) 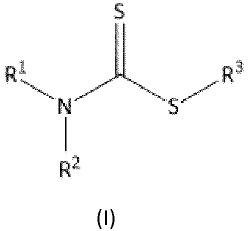 wherein
R¹ represents a first substituent having from 1 to 8 carbon atoms and comprising at least one functional group selected from alcohols, amines, ethers, ketones, acetals, ketals, aminoacetals, hemiaminal ethers or combinations thereof, preferably selected from alcohols, amines, ethers or combinations thereof, wherein R¹ comprises at most two amine functional groups;
and
R² represents H or a second substituent having from 1 to 8 carbon atoms and optionally comprising at least one functional group selected from alcohols, amines, ethers, ketones, acetals, ketals, aminoacetals, hemiaminal ethers or combinations thereof, preferably selected from alcohols, amines, ethers or combinations thereof;
or
R¹ and R² are connected to form a heterocyclic 3 to 7 membered ring comprising at least 2 heteroatoms, which is optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from H and C₁-C₄ alkyl;
and
R³ represents hydrogen or a cation;
with the provisio that when R¹ comprises no other functional groups than alcohols, the process does not comprise the use of a second depressant selected from polymers comprising an allyl thiourea functional group and a hydrophilic acrylamide group;
**characterized in that** recovering the froth in step (d) provides a second pulp comprising the first mineral which has an increased concentration of the first mineral based on dry weight, compared to the pulp provided in step (a).

2. A process for recovering a first mineral comprising the steps of:
(a) providing a pulp comprising solids and water, wherein the solids comprise the first mineral;
(b) adding a first depressant to the pulp;
(c) subjecting the pulp to a froth flotation process to produce a froth comprising the first mineral; and
(d) recovering the froth;
**characterized in that** the first mineral is a sulfide mineral, and the first depressant consists of one or more dithiocarbamic acids or salts thereof of formula (I) 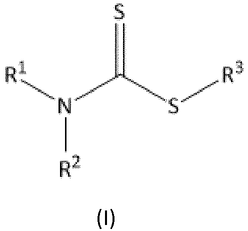 wherein
R¹ represents a first substituent having from 1 to 8 carbon atoms and comprising at least one functional group selected from alcohols, amines, ethers, ketones, acetals, ketals, aminoacetals, hemiaminal ethers or combinations thereof, preferably selected from alcohols, amines, ethers or combinations thereof, wherein R¹ comprises at most two amine functional groups;
and
R² represents H or a second substituent having from 1 to 8 carbon atoms and optionally comprising at least one functional group selected from alcohols, amines, ethers, ketones, acetals, ketals, aminoacetals, hemiaminal ethers or combinations thereof, preferably selected from alcohols, amines, ethers or combinations thereof;
or
R¹ and R² are connected to form a heterocyclic 3 to 7 membered ring comprising at least 2 heteroatoms, which is optionally substituted with one, two or three functional groups selected from -OH, C₁-C₄ alkyl, C₂-C₄ alkenyl, C₁-C₄ hydroxyalkyl, -NR^{a}R^{b} and combinations thereof, wherein R^{a} and R^{b} are independently selected from Hand C₁-C₄ alkyl;
and
R³ represents hydrogen or a cation;
with the provisio that when R¹ comprises no other functional groups than alcohols, the process does not comprise the use of a second depressant selected from polymers comprising an allyl thiourea functional group and a hydrophilic acrylamide group;
with the provisio that the first depressant is not N-(2-aminoethyl)dithiocarbamic acid or a salt thereof.

3. The process of claim 1 or 2 wherein R¹ represents a first substituent having from 2 to 5 carbon atoms and comprising one or two functional groups selected from amines, ethers or combinations thereof.

4. The process of any one of claims 1-3 wherein
• R¹ represents a first substituent having from 2 to 5 carbon atoms and comprising one or two alcohols; or
• R¹ represents a first substituent having from 2 to 5 carbon atoms and comprising one secondary or tertiary amine, preferably R¹ represents a first substituent having from 4 to 5 carbon atoms and comprising one tertiary amine.

5. The process of any one of claims 1-4, preferably of claim 4 wherein
• R¹ and R² are identical; or
• R² represents H, phenyl or a C₁-C₅ alkyl, preferably R² represents H, phenyl or a C₁-C₂ alkyl.

6. The process of claim 1 or 2 wherein R¹ and R² are connected to form a heterocyclic 5 or 6 membered ring comprising 2 heteroatoms selected from N and O, which is optionally substituted with a single functional group selected from - C₁-C₄ alkyl, preferably the heterocyclic ring is substituted with a single methyl group.

7. The process of claim 1 wherein the dithiocarbamic acids or salts thereof of formula (I) are selected from the group consisting of compounds 1,3-20 acid as defined in the following table or a salt thereof, preferably compounds 1, 3-6, 8-10, 12-20 acid as defined in the following table, or a salt thereof, more preferably compound 1, 3, 8, 9 acid as defined in the following table, or a salt thereof:
| **Compound name** | **Compound Structure** | **CAS** | **Systematic name** |
|---|---|---|---|
| diethanolamine dithiocarbamic acid | 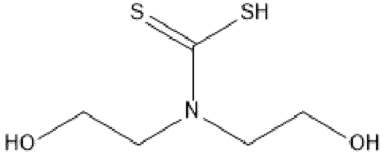 | 1528-72-9 | Bis(2-hydroxyethyl)carba modithioic acid |
| (**compound 1 acid**) | | | |
| 3-amino-1,2-propanediol dithiocarbamic acid | 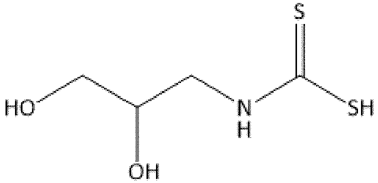 | NA | 2,3-dihydroxypropylcarb amodithioic acid |
| (**compound 3 acid**) | | | |
| 2-(methylamino)etha nol dithiocarbamic acid | 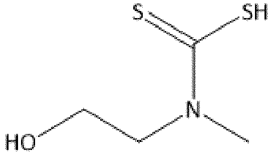 | 91308-51-9 | methyl(2-hydroxyethyl)carba modithioic acid |
| (**compound 4 acid**) | | | |
| bis(2-hydroxypropyl)ami ne dithiocarbamic acid | 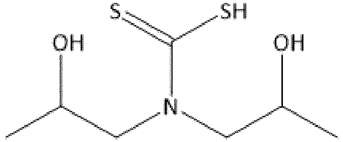 | 144796-43-0 | Bis(2-hydroxypropyl)carba modithioic acid |
| (**compound 5 acid**) | | | |
| bis(2-methoxyethyl)ami ne dithiocarbamic acid | 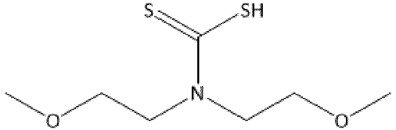 | 395653-20-0 | Bis(2-methoxyethyl)carba modithioic acid |
| (**compound 6 acid**) | | | |
| 2-amino-2-methyl-1,3,-propanediol dithiocarbamic acid | 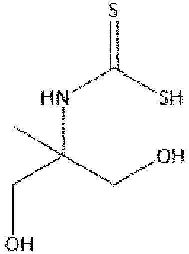 | NA | 2-amino-2-methyl-1,3,-propanediol carbamodithioic acid |
| (**compound 7 acid**) | | | |
| morpholine dithiocarbamic acid | 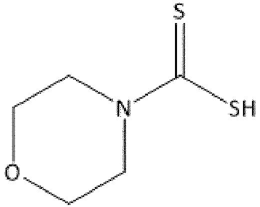 | 3581-30-4 | 4-Morpholinecarbodit hioic acid |
| (**compound 8 acid**) | | | |
| N-methylpiperazine dithiocarbamic acid | 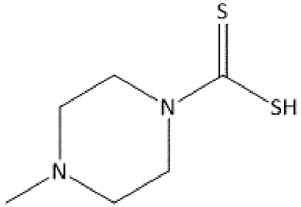 | 5430-77-3 | 4-Methyl-1-piperazinecarbodithi oic acid |
| (**compound 9 acid**) | | | |
| N-(2-hydroxyethyl)anilin e dithiocarbamic acid | 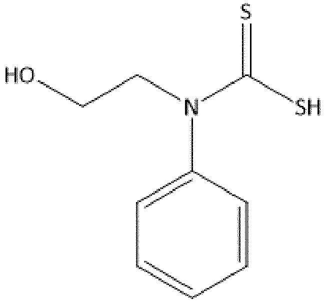 | 1006655-77-1 | phenyl(2-hydroxyethyl)carba modithioic acid |
| (**compound 10 acid**) | | | |
| N-phenylethylenedia mine dithiocarbamic acid | 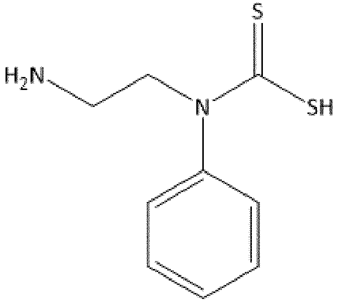 | NA | phenyl(2-aminoethyl)carbam odithioic acid |
| (**compound 11 acid**) | | | |
| (2-methoxyethyl)met hylamine dithiocarbamic acid | 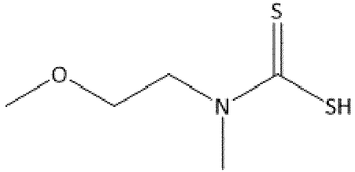 | 884739-17-7 | methyl(2-methoxyethyl)carba modithioic acid |
| (**compound 12 acid**) | | | |
| 3,3'-iminobis(N,N-dimethylpropylami ne) dithiocarbamic acid | 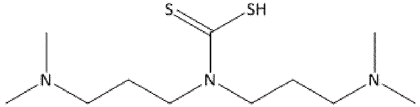 | 1161069-75-5 | *Bis*[(3-(dimethylamino)pro pyl]carbamodithioic acid |
| (**compound 13 acid**) | | | |
| 2-(ethylamino)ethan ol dithiocarbamic acid | 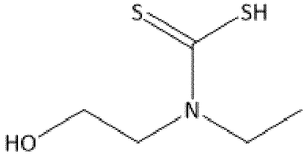 | 91308-52-0 | Ethyl(2-hydroxyethyl)carba modithioic acid |
| (**compound 14 acid**) | | | |
| 2-(isopropylamino)et hanol dithiocarbamic acid | 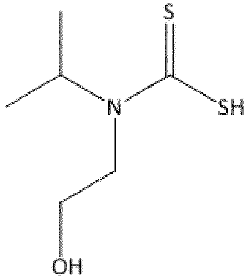 | 1442373-54-7 | isopropyl(2-hydroxyethyl)carba modithioic acid |
| (**compound 15 acid**) | | | |
| ethanolamine dithiocarbamic acid | 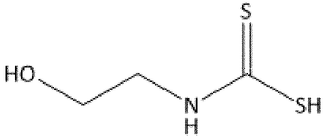 | 59333-68-5 | 2-hydroxyethylcarbam odithioic acid |
| (**compound 16 acid**) | | | |
| sodium N,N'-Dimethylethylened iamine dithiocarbamic acid | 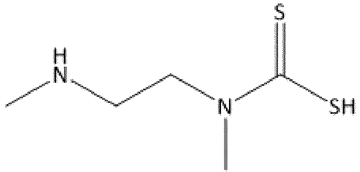 | 58708-60-4 | methyl (2-N-methylaminoethyl)carbam odithioic acid |
| (**compound 17 acid**) | | | |
| piperazine dithiocarbamic acid | 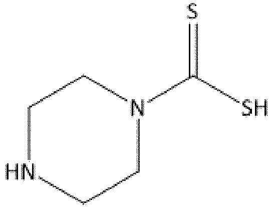 | 99-00-3 | 1-Piperazinecarbodithi oic acid |
| (**compound 18 acid**) | | | |
| ethylenediamine dithiocarbamic acid | 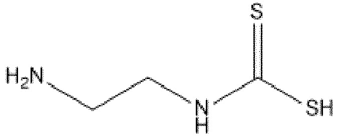 | 20950-84-9 | (2-Aminoethyl)carbam odithioic acid |
| (**compound 19 acid**) | | | |
| imidazole dithiocarbamic acid | 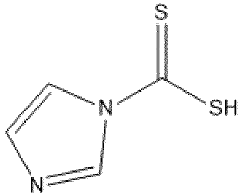 | 732930-06-2 | 1H-Imidazole-1-carbodithioic acid |
| (**compound 20 acid**) | | | |

8. The process of claim 2 wherein the dithiocarbamic acids or salts thereof of formula (I) are selected from the group consisting of compounds 1,3-18, 20 acid as defined in the following table or a salt thereof, preferably compounds 1, 3-6, 8-10, 12-18, 20 acid as defined in the following table, or a salt thereof, more preferably compound 1, 3, 8, 9 acid as defined in the following table, or a salt thereof:
| **Compound name** | **Compound Structure** | **CAS** | **Systematic name** |
|---|---|---|---|
| diethanolamine dithiocarbamic acid | 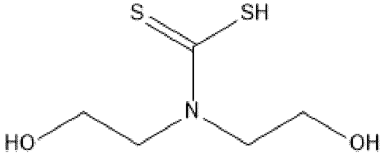 | 1528-72-9 | Bis(2-hydroxyethyl)carba modithioic acid |
| (**compound 1 acid**) | | | |
| 3-amino-1,2-propanediol dithiocarbamic acid | 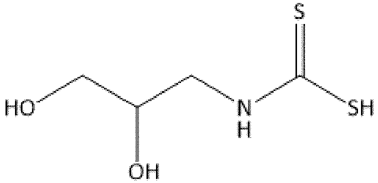 | NA | 2,3-dihydroxypropylcarb amodithioic acid |
| (**compound 3 acid**) | | | |
| 2-(methylamino)etha nol dithiocarbamic acid | 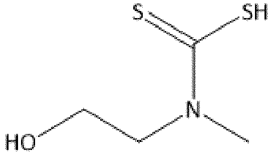 | 91308-51-9 | methyl(2-hydroxyethyl)carba modithioic acid |
| (**compound 4 acid**) | | | |
| bis(2-hydroxypropyl)ami ne dithiocarbamic acid | 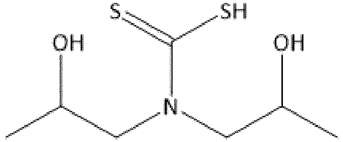 | 144796-43-0 | Bis(2-hydroxypropyl)carba modithioic acid |
| (**compound 5 acid**) | | | |
| bis(2-methoxyethyl)ami ne dithiocarbamic acid | 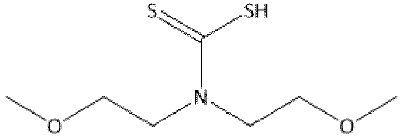 | 395653-20-0 | Bis(2-methoxyethyl)carba modithioic acid |
| (**compound 6 acid**) | | | |
| 2-amino-2-methyl-1,3,-propanediol dithiocarbamic acid | 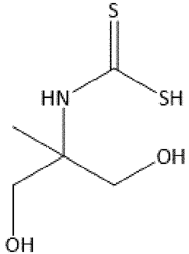 | NA | 2-amino-2-methyl-1,3,-propanediol carbamodithioic acid |
| (**compound 7 acid**) | | | |
| morpholine dithiocarbamic acid | 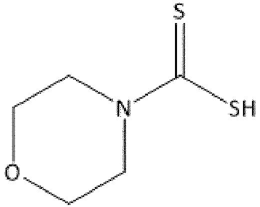 | 3581-30-4 | 4-Morpholinecarbodit hioic acid |
| (**compound 8 acid**) | | | |
| N-methylpiperazine dithiocarbamic acid | 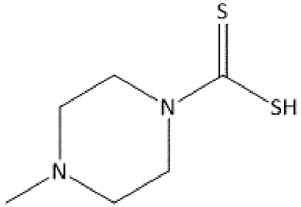 | 5430-77-3 | 4-Methyl-1-piperazinecarbodithi oic acid |
| (**compound 9 acid**) | | | |
| N-(2-hydroxyethyl)anilin e dithiocarbamic acid | 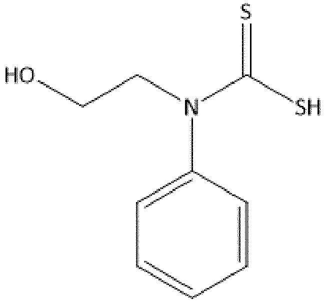 | 1006655-77-1 | phenyl(2-hydroxyethyl)carba modithioic acid |
| (**compound 10 acid**) | | | |
| N-phenylethylenedia mine dithiocarbamic acid | 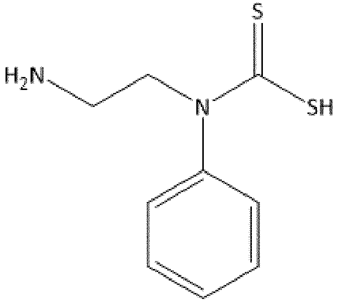 | NA | phenyl(2-aminoethyl)carbam odithioic acid |
| (**compound 11 acid**) | | | |
| (2-methoxyethyl)met hylamine dithiocarbamic acid | 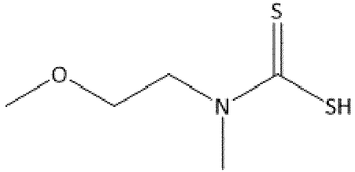 | 884739-17-7 | methyl(2-methoxyethyl)carba modithioic acid |
| (**compound 12 acid**) | | | |
| 3,3'-iminobis(N,N-dimethylpropylami ne) dithiocarbamic acid | 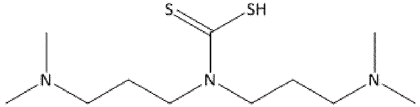 | 1161069-75-5 | *Bis*[(3-(dimethylamino)pro pyl]carbamodithioic acid |
| (**compound 13 acid**) | | | |
| 2-(ethylamino)ethan ol dithiocarbamic acid | 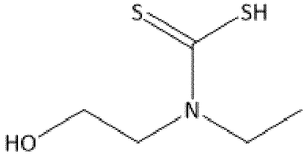 | 91308-52-0 | Ethyl(2-hydroxyethyl)carba modithioic acid |
| (**compound 14 acid**) | | | |
| 2-(isopropylamino)et hanol dithiocarbamic acid | 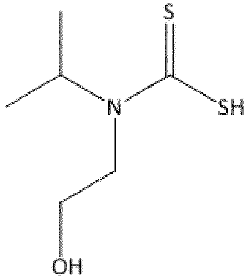 | 1442373-54-7 | isopropyl(2-hydroxyethyl)carba modithioic acid |
| (**compound 15 acid**) | | | |
| ethanolamine dithiocarbamic acid | 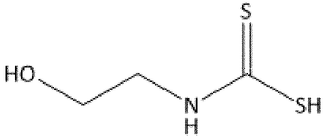 | 59333-68-5 | 2-hydroxyethylcarbam odithioic acid |
| (**compound 16 acid**) | | | |
| sodium N,N'-Dimethylethylened iamine dithiocarbamic acid | 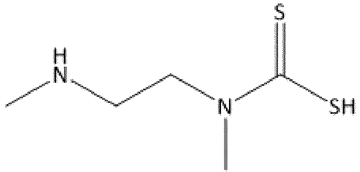 | 58708-60-4 | methyl (2-N-methylaminoethyl)carbam odithioic acid |
| (**compound 17 acid**) | | | |
| piperazine dithiocarbamic acid | 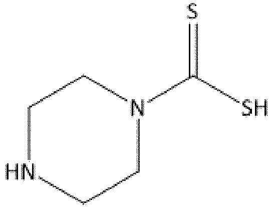 | 99-00-3 | 1-Piperazinecarbodithi oic acid |
| (**compound 18 acid**) | | | |
| imidazole dithiocarbamic acid | 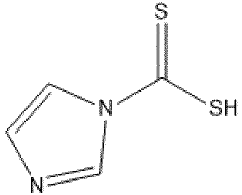 | 732930-06-2 | 1H-Imidazole-1-carbodithioic acid |
| (**compound 20 acid**) | | | |

9. The process of any one of claims 1-8 wherein the first mineral is a molybdenum sulfide mineral, preferably the first mineral is molybdenite and wherein the solids comprising the first mineral further comprises a second mineral and the process comprises depressing the second mineral into the pulp.

10. The process of claim 9 wherein the second mineral is a sulfide mineral, preferably a sulfide mineral selected from the group consisting of Chalcopyrite, Chalcocite, Covellite, Pyrite, Bornite, Sphalerite, and combinations thereof, preferably Chalcopyrite.

11. The process of any one of claims 1-10, preferably of claim 10 wherein the pulp provided in step (a) further comprises a collector, preferably a collector selected from the group consisting of xanthates, xanthogen formates, thioureas, thionocarbamates, (di)thiophosphates, dithiophosphinates, N-alkoxycarbonyl dithiocarbamates, dialkyldithiocarbamates, mercaptobenzothioazoles, nitriles and combinations thereof.

12. The process of any one of claims 1-11 further comprising the step of:
(b2) adding a second depressant to the pulp provided in step (a);
wherein step (b2) may be performed before, during and/or after step (b) and preferably the second depressant is NaHS.

13. A composition comprising the first depressant as described in any one of claims 1, 3-7 and further comprising a sulfide mineral, preferably a sulfide mineral selected from the group consisting of Molybdenite, Chalcopyrite, Chalcocite, Covellite, Pyrite, Bornite, Sphalerite, and combinations thereof, preferably selected from the group consisting of Molybdenite and Chalcopyrite; with the provisio that the first depressant is not *N*-(2-Aminoethyl)carbamodithioic acid or a salt thereof or a compound of formula (I) wherein R¹ comprises no other functional groups than alcohols.

14. A composition comprising a first depressant and further comprising one, two or three of:
• a collector as described in claim 11;
• a second depressant as described in claim 12; and
• a mineral;
wherein the first depressant consists of one or more dithiocarbamic acids or salts thereof of formula (I) as described in claim 1,
wherein
R¹ represents a first substituent having from 2 to 5 carbon atoms and comprising one or two alcohols; or
R¹ and R² are identical; or
R¹ and R² are connected to form a heterocyclic 5 or 6 membered ring comprising 2 heteroatoms selected from N and O, which is optionally substituted with a single functional group selected from - C₁-C₄ alkyl, preferably the heterocyclic ring is substituted with a single methyl group; or the dithiocarbamic acids or salts thereof of formula (I) are selected from the group consisting of compounds 1,3-18, 20 acid as defined in claim 8 or a salt thereof,
with the provisio that the first depressant is not a compound of formula (I) wherein R¹ comprises no other functional groups than alcohols.

15. The composition of claim 13 or 14 which is a mining additive composition comprising a second depressant as described in claim 12 in an amount of at least 5 wt.%, based on total weight of the additive composition excluding solvents, preferably at least 10 wt.%, based on total weight of the additive composition excluding solvents; wherein the first depressant is preferably present in an amount of at least 5 wt.%, based on total weight of the additive composition excluding solvents, preferably at least 20 wt.%, based on total weight of the additive composition excluding solvents.

16. The composition of claim 13 or 14 which is a mineral pulp comprising a sulfide mineral, preferably a sulfide mineral selected from the group consisting of Molybdenite, Chalcopyrite, Chalcocite, Covellite, Pyrite, Bornite, Sphalerite, and combinations thereof, in an amount of at least 0.01 wt.%, based on total weight of the mineral pulp, preferably at least 0.1 wt.% and optionally further comprising
• a collector as described in claim 11 in an amount of at least 10 ppm w/w, based on total weight of mineral pulp; and/or
• a second depressant as described in claim 12 in an amount of at least 10 ppm, based on total weight of the mineral pulp;
wherein the first depressant is preferably present in an amount of at least 10 ppm, based on total weight of the mineral pulp.

17. Use of a dithiocarbamic acid or salt thereof of formula (I) as described in any one of claims 1-8 in the recovery, preferably the froth flotation recovery, of minerals with the provisio that the dithiocarbamic acid or salt thereof is not *N*-(2-Aminoethyl)carbamodithioic acid or a salt thereof or a compound of formula (I) wherein R¹ comprises no other functional groups than alcohols.

18. Use according to claim 17 in the froth flotation recovery of a sulfide mineral, preferably of Molybdenite.

19. Use according to claim 17 or 18 wherein the dithiocarbamic acid or salt thereof is a dithiocarbamic acid or salt thereof of formula (I) as described in claim 1 wherein
R¹ represents a first substituent having from 2 to 5 carbon atoms and comprising one or two alcohols; or
R¹ and R² are identical; or
R¹ and R² are connected to form a heterocyclic 5 or 6 membered ring comprising 2 heteroatoms selected from N and O, which is optionally substituted with a single functional group selected from - C₁-C₄ alkyl, preferably the heterocyclic ring is substituted with a single methyl group; or the dithiocarbamic acids or salts thereof of formula (I) are selected from the group consisting of compounds 1,3-18, 20 acid as defined in claim 8 or a salt thereof.

20. A dithiocarbamic acid or salt thereof which is
• compound 3 acid (2,3-dihydroxypropylcarbamodithioic acid) as defined in the following table or a salt thereof,
• compound 7 acid (2-amino-2-methyl-1,3,-propanediol carbamodithioic acid) as defined in the following table or a salt thereof, or
• compound 11 acid (phenyl(2-aminoethyl)carbamodithioic acid) as defined in the following table or a salt thereof:
| **Compound name** | **Compound Structure** | **CAS** | **Systematic name** |
|---|---|---|---|
| 3-amino-1,2-propanediol dithiocarbamic acid (**compound 3 acid**) | 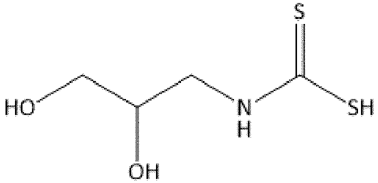 | NA | 2,3-dihydroxypropylcarb amodithioic acid |
| 2-amino-2-methyl-1,3,-propanediol dithiocarbamic acid (**compound 7 acid**) | 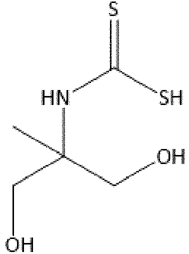 | NA | 2-amino-2-methyl-1,3,-propanediol carbamodithioic acid |
| N-phenylethylenedia mine dithiocarbamic acid (**compound 11 acid**) | 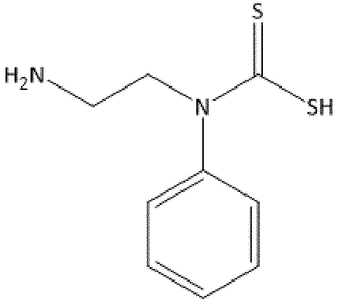 | NA | phenyl(2-aminoethyl)carbam odithioic acid |

21. Use of the dithiocarbamic acid or salt thereof of formula (I) of claim 20 in the recovery, preferably the froth flotation recovery, of minerals.

## Patentansprüche

1. Verfahren zur Rückgewinnung eines ersten Minerals, umfassend die folgenden Schritte:
(a) Bereitstellen einer Pulpe, die Feststoffe und Wasser umfasst, wobei die Feststoffe das erste Mineral umfassen;
(b) Zugeben eines ersten Depressants zu der Pulpe;
(c) Aussetzen der Pulpe einem Schaumflotationsverfahren, um einen Schaum herzustellen, der das erste Mineral umfasst; und
(d) Rückgewinnen des Schaums;
wobei das erste Mineral ein Sulfidmineral ist und das erste Depressant aus einer oder mehreren Dithiocarbamidsäuren oder Salzen davon der Formel (1) besteht 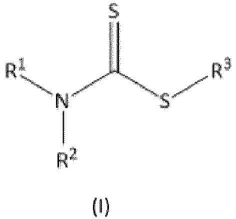
wobei
R¹ einen ersten Substituenten darstellt, der 1 bis 8 Kohlenstoffatome aufweist und mindestens eine funktionelle Gruppe umfasst, die aus Alkoholen, Aminen, Ethern, Ketonen, Acetalen, Ketalen, Aminoacetalen, halbaminalen Ethern oder Kombinationen davon ausgewählt ist, vorzugsweise ausgewählt aus Alkoholen, Aminen, Ethern oder Kombinationen davon, wobei R¹ höchstens zwei funktionelle Amingruppen umfasst;
und
R² H oder einen zweiten Substituenten darstellt, der 1 bis 8 Kohlenstoffatome aufweist und optional mindestens eine funktionelle Gruppe umfasst, die aus Alkoholen, Aminen, Ethern, Ketonen, Acetalen, Ketalen, Aminoacetalen, halbaminalen Ethern oder Kombinationen davon ausgewählt ist, vorzugsweise ausgewählt aus Alkoholen, Aminen, Ethern oder Kombinationen davon;
oder
R¹ und R² verbunden sind, um einen heterocyclischen 3- bis 7-gliedrigen Ring zu bilden, der mindestens 2 Heteroatome umfasst, der optional mit einer, zwei oder drei funktionellen Gruppen substituiert ist, die aus -OH, C₁-C₄-Alkyl, C₂-C₄-Alkenyl, C₁-C₄-Hydroxyalkyl, -NR^{a}R^{b} und Kombinationen davon ausgewählt sind, wobei R^{a} und R^{b} unabhängig voneinander aus H und C₁-C₄-Alkyl ausgewählt sind;
und
R³ Wasserstoff oder ein Kation darstellt;
mit der Maßgabe, dass, wenn R¹ keine anderen funktionellen Gruppen als Alkohole umfasst, das Verfahren nicht die Verwendung eines zweiten Depressants umfasst, das aus Polymeren ausgewählt ist, die eine funktionelle Allylthioharnstoffgruppe und eine hydrophile Acrylamidgruppe umfassen;
**dadurch gekennzeichnet, dass** die Rückgewinnung des Schaums in Schritt (d) eine zweite Pulpe bereitstellt, die das erste Mineral umfasst, die eine erhöhte Konzentration des ersten Minerals auf Basis des Trockengewichts im Vergleich zu der in Schritt (a) bereitgestellten Pulpe aufweist.

2. Verfahren zur Rückgewinnung eines ersten Minerals, umfassend die folgenden Schritte:
(a) Bereitstellen einer Pulpe, die Feststoffe und Wasser umfasst, wobei die Feststoffe das erste Mineral umfassen;
(b) Zugeben eines ersten Depressants zu der Pulpe;
(c) Aussetzen der Pulpe einem Schaumflotationsverfahren, um einen Schaum herzustellen, der das erste Mineral umfasst; und
(d) Rückgewinnen des Schaums;
**dadurch gekennzeichnet, dass** das erste Mineral ein Sulfidmineral ist und das erste Depressant aus einer oder mehreren Dithiocarbamidsäuren oder Salzen davon der Formel (1) besteht 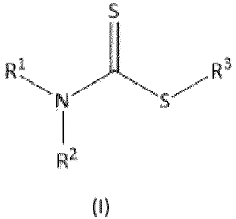
wobei
R¹ einen ersten Substituenten darstellt, der 1 bis 8 Kohlenstoffatome aufweist und mindestens eine funktionelle Gruppe umfasst, die aus Alkoholen, Aminen, Ethern, Ketonen, Acetalen, Ketalen, Aminoacetalen, halbaminalen Ethern oder Kombinationen davon ausgewählt ist, vorzugsweise ausgewählt aus Alkoholen, Aminen, Ethern oder Kombinationen davon, wobei R¹ höchstens zwei funktionelle Amingruppen umfasst;
und
R² H oder einen zweiten Substituenten darstellt, der 1 bis 8 Kohlenstoffatome aufweist und optional mindestens eine funktionelle Gruppe umfasst, die aus Alkoholen, Aminen, Ethern, Ketonen, Acetalen, Ketalen, Aminoacetalen, halbaminalen Ethern oder Kombinationen davon ausgewählt ist, vorzugsweise ausgewählt aus Alkoholen, Aminen, Ethern oder Kombinationen davon;
oder
R¹ und R² verbunden sind, um einen heterocyclischen 3- bis 7-gliedrigen Ring zu bilden, der mindestens 2 Heteroatome umfasst, der optional mit einer, zwei oder drei funktionellen Gruppen substituiert ist, die aus -OH, C₁-C₄-Alkyl, C₂-C₄-Alkenyl, C₁-C₄-Hydroxyalkyl, -NR^{a}R^{b} und Kombinationen davon ausgewählt sind, wobei R^{a} und R^{b} unabhängig voneinander aus H und C₁-C₄-Alkyl ausgewählt sind;
und
R³ Wasserstoff oder ein Kation darstellt;
mit der Maßgabe, dass, wenn R¹ keine anderen funktionellen Gruppen als Alkohole umfasst, das Verfahren nicht die Verwendung eines zweiten Depressants umfasst, das aus Polymeren ausgewählt ist, die eine funktionelle Allylthioharnstoffgruppe und eine hydrophile Acrylamidgruppe umfassen;
mit der Maßgabe, dass das erste Depressant nicht N-(2-Aminoethyl)dithiocarbaminsäure oder ein Salz davon ist.

3. Verfahren nach Anspruch 1 oder 2, wobei R¹ einen ersten Substituenten darstellt, der 2 bis 5 Kohlenstoffatome aufweist und eine oder zwei funktionelle Gruppen umfasst, die aus Aminen, Ethern oder Kombinationen davon ausgewählt sind.

4. Verfahren nach einem der Ansprüche 1 - 3, wobei
• R¹ einen ersten Substituenten darstellt, der 2 bis 5 Kohlenstoffatome aufweist und einen oder zwei Alkohole umfasst; oder
• R¹ einen ersten Substituenten darstellt, der 2 bis 5 Kohlenstoffatome aufweist und ein sekundäres oder tertiäres Amin umfasst, wobei R¹ vorzugsweise einen ersten Substituenten mit 4 bis 5 Kohlenstoffatomen darstellt und ein tertiäres Amin umfasst.

5. Verfahren nach einem der Ansprüche 1 - 4, vorzugsweise nach Anspruch 4, wobei
• R¹ und R² identisch sind; oder
• R² für H, Phenyl oder ein C₁-C₅-Alkyl steht, wobei R² vorzugsweise für H, Phenyl oder ein C₁-C₂-Alkyl steht.

6. Verfahren nach Anspruch 1 oder 2, wobei R¹ und R² verbunden sind, um einen heterocyclischen 5- oder 6-gliedrigen Ring zu bilden, umfassend 2 Heteroatome, ausgewählt aus N und O, der optional mit einer einzelnen funktionellen Gruppe substituiert ist, ausgewählt aus -C₁-C₄-Alkyl, wobei der heterocyclische Ring vorzugsweise mit einer einzelnen Methylgruppe substituiert ist.

7. Verfahren nach Anspruch 1, wobei die Dithiocarbaminsäuren oder Salze davon der Formel (I) aus der Gruppe ausgewählt sind, die aus den Verbindungen 1,3-20-Säure, wie in der folgenden Tabelle definiert, oder einem Salz davon, vorzugsweise den Verbindungen 1, 3-6, 8-10, 12-20-Säure, wie in der folgenden Tabelle definiert, oder einem Salz davon, bevorzugter Verbindung 1, 3, 8, 9-Säure, wie in der folgenden Tabelle definiert, oder einem Salz davon besteht:
| **Verbindungsname** | **Verbindungsstruktur** | **CAS** | **Systematischer Name** |
|---|---|---|---|
| Diethanolamindithiocarbamidsäure (**Verbindung 1 Säure**) | 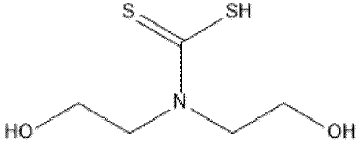 | 1528-72-9 | Bis(2-hydroxyethyl)-carbamodithiosäure |
| 3-Amino-1,2-propandioldithiocarbaminsäure (**Verbindung 3 Säure**) | 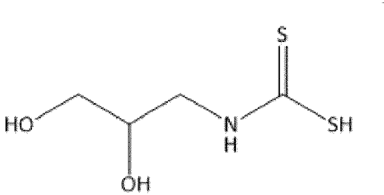 | NA | 2,3-Dihydroxypropylcarbamodithiosäure |
| 2-(Methylamino)-ethanoldithiocarbaminsäure (**Verbindung 4 Säure**) | 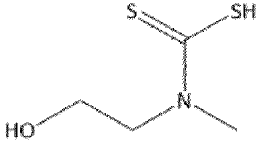 | 91308-51-9 | Methyl(2-hydroxyethyl)-carbamodithiosäure |
| Bis(2-hydroxypropyl)-aminodithiocarbaminsäure **(Verbindung 5 Säure)** | 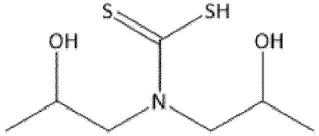 | 144796-43-0 | Bis(2-hydroxypropyl)-carbamodithiosäure |
| Bis(2-methoxyethyl)-aminodithiocarbaminsäure (**Verbindung 6 Säure**) | 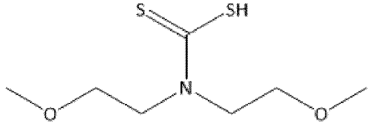 | 395653-20-0 | Bis(2-methoxyethyl)-carbamodithiosäure |
| 2-Amino-2-methyl-1,3-propandioldithiocarbaminsäure (**Verbindung 7 Säure**) | 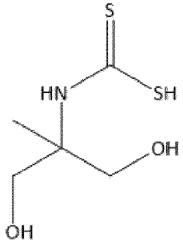 | NA | 2-Amino-2-methyl-1,3-propandiolcarbamodithiosäure |
| Morpholindithiocarbaminsäure (**Verbindung 8 Säure**) | 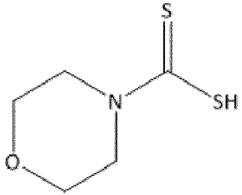 | 3581-30-4 | 4-Morpholincarbodithiosäure |
| N-Methylpiperazindithiocarbaminsäure (**Verbindung 9 Säure**) | 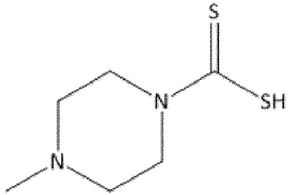 | 5430-77-3 | 4-Methyl-1-piperazincarbodithiosäure |
| N-(2-Hydroxyethyl)anilin-dithiocarbaminsäure (**Verbindung 10 Säure**) | 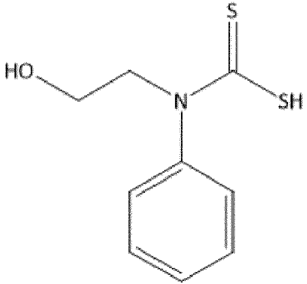 | 1006655-77-1 | Phenyl(2-hydroxyethyl)-carbamodithiosäure |
| N-Phenylethylendiamindithiocarbaminsäure (**Verbindung 11 Säure**) | 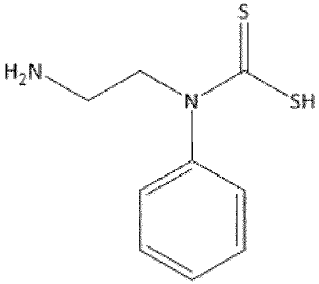 | NA | Phenyl(2-aminoethyl)-carbamodithiosäure |
| (2-Methoxyethyl)methylamin-dithiocarbaminsäure (**Verbindung 12 Säure**) | 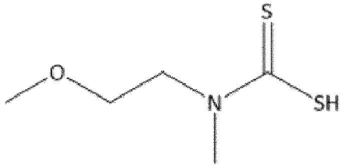 | 884739-17-7 | Methyl(2-methoxyethyl)-carbamodithiosäure |
| 3,3'-Iminobis(N,N-dimethylpropylamin)-dithiocarbaminsäure **(Verbindung 13 Säure)** | 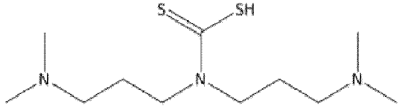 | 1161069-75-5 | Bis[3-(Dimethylamino)-propyl]carbamodithiosäure |
| 2-(Ethylamino)ethanol-dithiocarbaminsäure **(Verbindung 14 Säure)** | 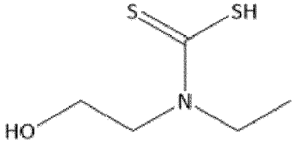 | 91308-52-0 | Ethyl(2-hydroxyethyl)-carbamodithiosäure |
| 2-(Isopropylamino)-ethanoldithiocarbaminsäure **(Verbindung 15 Säure)** | 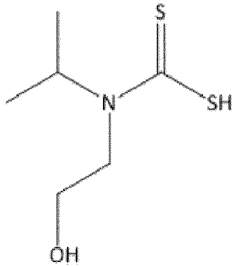 | 1442373-54-7 | Isopropyl(2-hydroxyethyl)-carbamodithiosäure |
| Ethanolamindithiocarbaminsäure **(Verbindung 16 Säure)** | 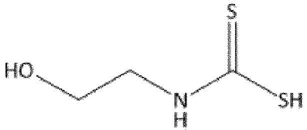 | 59333-68-5 | 2-Hydroxyethylcarbamodithiosäure |
| Natrium-N,N'-dimethylethylendiamindithiocarbaminsäure **(Verbindung 17 Säure)** | 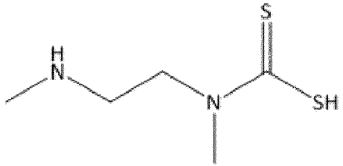 | 58708-60-4 | Methyl(2-N-methylaminoethyl)carbamodithiosäure |
| Piperazindithiocarbaminsäure **(Verbindung 18 Säure)** | 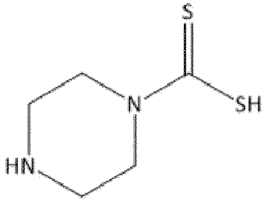 | 99-00-3 | 1-Piperazincarbodithiosäure |
| Ethylendiamindithiocarbaminsäure **(Verbindung 19 Säure)** | 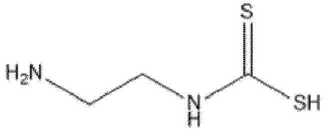 | 20950-84-9 | (2-Aminoethyl)-carbodithiosäure |
| Imidazoldithiocarbaminsäure **(Verbindung 20 Säure)** | 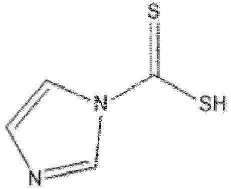 | 732930-06-2 | 1H-Imidazol-1-carbodithiosäure |

8. Verfahren nach Anspruch 2, wobei die Dithiocarbaminsäuren oder Salze davon der Formel (I) aus der Gruppe ausgewählt sind, die aus den Verbindungen 1, 3-18, 20-Säuren, wie in der folgenden Tabelle definiert, oder einem Salz davon, vorzugsweise den Verbindungen 1, 3-6, 8-10, 12-18, 20-Säuren, wie in der folgenden Tabelle definiert, oder einem Salz davon, bevorzugter einer Verbindung 1, 3, 8, 9-Säure, wie in der folgenden Tabelle definiert, oder einem Salz davon, besteht:
| **Verbindungsname** | **Verbindungsstruktur** | **CAS** | **Systematischer Name** |
|---|---|---|---|
| Diethanolamindithiocarbamidsäure **(Verbindung 1 Säure)** | 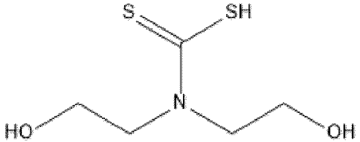 | 1528-72-9 | Bis(2-hydroxyethyl)-carbamodithiosäure |
| 3-Amino-1,2-propandioldithiocarbaminsäure **(Verbindung 3 Säure)** | 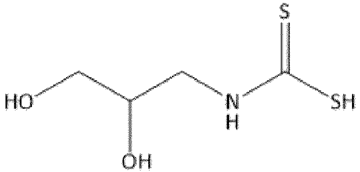 | NA | 2,3-Dihydroxypropylcarbamodithiosäure |
| 2-(Methylamino)-ethanoldithio-carbaminsäure **(Verbindung 4 Säure)** | 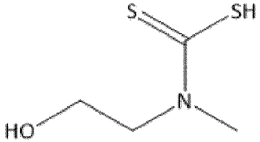 | 91308-51-9 | Methyl(2-hydroxyethyl)-carbamodithiosäure |
| Bis(2-hydroxypropyl)-aminodithio-carbaminsäure **(Verbindung 5 Säure)** | 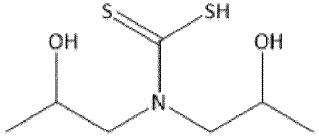 | 144796-43-0 | Bis(2-hydroxypropyl)-carbamodithiosäure |
| Bis(2-methoxyethyl)-aminodithio-carbaminsäure **(Verbindung 6 Säure)** | 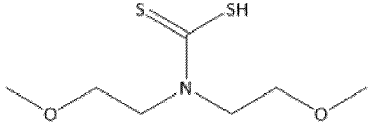 | 395653-20-0 | Bis(2-methoxyethyl)-carbamodithiosäure |
| 2-Amino-2-methyl-1,3-propandioldi-thiocarbaminsäure **(Verbindung 7 Säure)** | 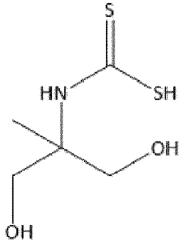 | NA | 2-Amino-2-methyl-1,3-propandiol-carbamodithiosäure |
| Morpholin-dithiocarbaminsäure **(Verbindung 8 Säure)** | 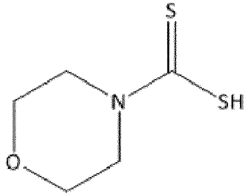 | 3581-30-4 | 4-Morpholin-carbo-dithiosäure |
| N-Methyl-piperazin-dithiocarbaminsäure **(Verbindung 9 Säure)** | 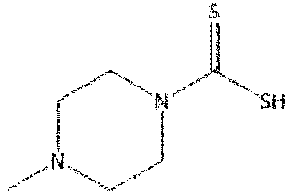 | 5430-77-3 | 4-Methyl-1-piperazin-carbo-dithiosäure |
| N-(2-Hydroxyethyl)anilin-dithiocarbaminsäure **(Verbindung 10 Säure)** | 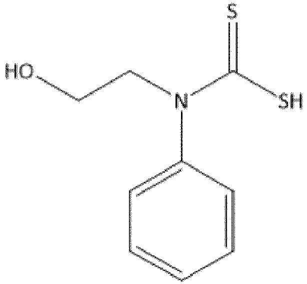 | 1006655-77-1 | Phenyl(2-hydroxyethyl)-carbamodithiosäure |
| N-Phenylethylen-diamin-dithiocarbaminsäure **(Verbindung 11 Säure)** | 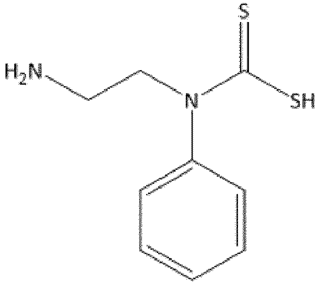 | NA | Phenyl(2-aminoethyl)-carbamodithiosäure |
| (2-Methoxyethyl)methylamin-dithiocarbaminsäure **(Verbindung 12 Säure)** | 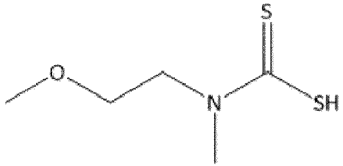 | 884739-17-7 | Methyl(2-methoxyethyl)-carbamodithiosäure |
| 3,3'-Iminobis(N,N-dimethylpropylamin)-dithiocarbaminsäure **(Verbindung 13 Säure)** | 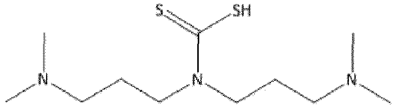 | 1161069-75-5 | Bis[3-(Dimethylamino)-propyl]carbamodithiosäure |
| 2-(Ethylamino)ethanoldithiocarbaminsäure **(Verbindung 14 Säure)** | 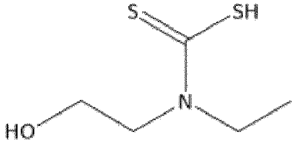 | 91308-52-0 | Ethyl(2-hydroxyethyl)-carbamodithiosäure |
| 2-(Isopropylamino)-ethanoldithiocarbaminsäure **(Verbindung 15 Säure)** | 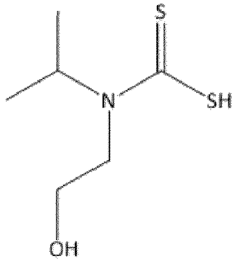 | 1442373-54-7 | Isopropyl(2-hydroxyethyl)-carbamodithiosäure |
| Ethanolamin-dithiocarbaminsäure **(Verbindung 16 Säure)** | 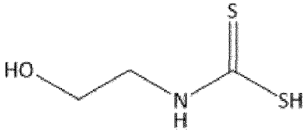 | 59333-68-5 | 2-Hydroxyethylcarbamodithiosäure |
| Natrium-N,N'-dimethylethylendiamin-dithiocarbaminsäure **(Verbindung 17 Säure)** | 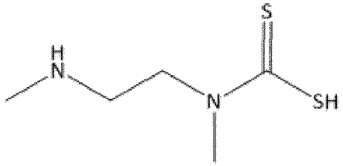 | 58708-60-4 | Methyl(2-N-methylaminoethyl)carbamodithiosäure |
| Piperazin-dithiocarbaminsäure **(Verbindung 18 Säure)** | 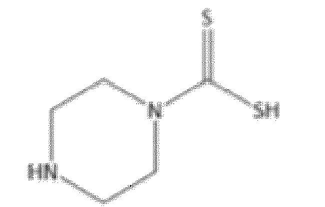 | 99-00-3 | 1-Piperazin-carbothiosäure |
| Imidazoldithio-carbaminsäure **(Verbindung 20 Säure)** | 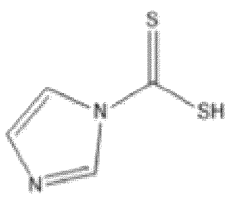 | 732930-06-2 | 1H-Imidazol-1-carbodithiosäure |

9. Verfahren nach einem der Ansprüche 1-8, wobei das erste Mineral ein Molybdänsulfidmineral ist, wobei das erste Mineral vorzugsweise Molybdänit ist, und wobei die Feststoffe, die das erste Mineral umfassen, weiter ein zweites Mineral umfassen, und das Verfahren das Eindrücken des zweiten Minerals in die Pulpe umfasst.

10. Verfahren nach Anspruch 9, wobei das zweite Mineral ein Sulfidmineral ist, vorzugsweise ein Sulfidmineral, das aus der Gruppe ausgewählt ist, die aus Chalkopyrit, Chalkosin, Covellin, Pyrit, Bornit, Sphalerit und Kombinationen davon besteht, vorzugsweise Chalkopyrit.

11. Verfahren nach einem der Ansprüche 1 bis 10, vorzugsweise nach Anspruch 10, wobei die in Schritt (a) bereitgestellte Pulpe weiter einen Kollektor umfasst, vorzugsweise einen Kollektor, der aus der Gruppe ausgewählt ist, die aus Xanthaten, Xanthogen-formiaten, Thioharnstoffen, Thionocarbamaten, (Di)thiophosphaten, Dithiophosphinaten, N-Alkoxycarbonyl-dithiocarbamaten, Dialkyldithiocarbamaten, Mercaptobenzothioazolen, Nitrilen und Kombinationen davon besteht.

12. Verfahren nach einem der Ansprüche 1-11 weiter umfassend den folgenden Schritt:
(b2) Zugabe eines zweiten Depressants zu der in Schritt (a) bereitgestellten Pulpe; wobei Schritt (b2) vor, während und/oder nach Schritt (b) durchgeführt werden kann und das zweite Depressant vorzugsweise NaHS ist.

13. Zusammensetzung, umfassend das erste Depressant, wie in einem der Ansprüche 1, 3-7 beschrieben, und weiter umfassend ein Sulfidmineral, vorzugsweise ein Sulfidmineral, ausgewählt aus der Gruppe, bestehend aus Molybdänit, Chalkopyrit, Chalkosin, Covellin, Pyrit, Bornit, Sphalerit und Kombinationen davon, vorzugsweise ausgewählt aus der Gruppe bestehend aus Molybdänit und Chalkopyrit;
mit der Maßgabe, dass das erste Depressant nicht *N*-(2-Aminoethyl)carbamodithiosäure oder ein Salz davon oder eine Verbindung der Formel (I) ist, wobei R¹ keine anderen funktionellen Gruppen als Alkohole umfasst.

14. Zusammensetzung, umfassend ein erstes Depressant und weiter umfassend eines, zwei oder drei von:
• einem Kollektor, wie in Anspruch 11 beschrieben;
• einem zweiten Depressant, wie in Anspruch 12 beschrieben; und
• einem Mineral;
wobei das erste Depressant aus einer oder mehreren Dithiocarbaminsäuren oder Salzen davon der Formel (I), wie in Anspruch 1 beschrieben, besteht,
wobei
R¹ einen ersten Substituenten mit 2 bis 5 Kohlenstoffatomen darstellt und ein oder zwei Alkohole umfasst; oder
R¹ und R² identisch sind; oder
R¹ und R² verbunden sind, um einen heterocyclischen 5- oder 6-gliedrigen Ring zu bilden, umfassend 2 Heteroatome, ausgewählt aus N und O, der optional mit einer einzelnen funktionellen Gruppe substituiert ist, ausgewählt aus -C₁-C₄-Alkyl, vorzugsweise ist der heterocyclische Ring mit einer einzelnen Methylgruppe substituiert; oder die Dithiocarbaminsäuren oder Salze davon der Formel (I) sind ausgewählt aus der Gruppe bestehend aus den Verbindungen 1, 3-18, 20 Säure, wie in Anspruch 8 definiert, oder ein Salz davon,
mit der Maßgabe, dass das erste Depressant keine Verbindung der Formel (I) ist, wobei R¹ keine anderen funktionellen Gruppen als Alkohole umfasst.

15. Zusammensetzung nach Anspruch 13 oder 14, die eine Additivzusammensetzung für den Bergbau ist, umfassend ein zweites Depressant, wie in Anspruch 12 beschrieben, in einer Menge von mindestens 5 Gew.-%, bezogen auf das Gesamtgewicht der Additivzusammensetzung ohne Lösungsmittel, vorzugsweise mindestens 10 Gew.-%, bezogen auf das Gesamtgewicht der Additivzusammensetzung ohne Lösungsmittel; wobei das erste Depressant vorzugsweise in einer Menge von mindestens 5 Gew.-% vorhanden ist, bezogen auf das Gesamtgewicht der Additivzusammensetzung ohne Lösungsmittel, vorzugsweise mindestens 20 Gew.-%, bezogen auf das Gesamtgewicht der Additivzusammensetzung ohne Lösungsmittel.

16. Zusammensetzung nach Anspruch 13 oder 14, die eine Mineralpulpe ist, umfassend ein Sulfidmineral, vorzugsweise ein Sulfidmineral, ausgewählt aus der Gruppe, bestehend aus Molybdänit, Chalkopyrit, Chalkosin, Covellin, Pyrit, Somit, Sphalerit und Kombinationen davon, in einer Menge von mindestens 0,01 Gew.-%, bezogen auf das Gesamtgewicht der Mineralpulpe, vorzugsweise mindestens 0,1 Gew.-%, und optional weiter umfassend
• einen Kollektor, wie in Anspruch 11 beschrieben, in einer Menge von mindestens 10 ppm w/w, bezogen auf das Gesamtgewicht der Mineralpulpe;
und/oder
• ein zweites Depressant, wie in Anspruch 12 beschrieben, in einer Menge von mindestens 10 ppm, bezogen auf das Gesamtgewicht der Mineralpulpe;
wobei das erste Depressant vorzugsweise in einer Menge von mindestens 10 ppm, bezogen auf das Gesamtgewicht der Mineralpulpe, vorhanden ist.

17. Verwendung einer Dithiocarbaminsäure oder eines Salzes davon der Formel (I) wie in einem der Ansprüche 1-8 beschrieben, zur Rückgewinnung, vorzugsweise zur Schaumflotationsrückgewinnung, von Mineralien mit der Maßgabe, dass die Dithiocarbaminsäure oder das Salz davon nicht *N*-(2-Aminoethyl)carbamodithiosäure oder ein Salz davon oder eine Verbindung der Formel (I) ist, wobei R¹ keine anderen funktionellen Gruppen als Alkohole umfasst.

18. Verwendung nach Anspruch 17 bei der Schaumflotationsrückgewinnung eines Sulfidminerals, vorzugsweise von Molybdänit.

19. Verwendung nach Anspruch 17 oder 18, wobei die Dithiocarbaminsäure oder ein Salz davon eine Dithiocarbaminsäure oder ein Salz davon der Formel (I) ist, wie in Anspruch 1 beschrieben, wobei
R¹ einen ersten Substituenten mit 2 bis 5 Kohlenstoffatomen darstellt und einen oder zwei Alkohole umfasst; oder
R¹ und R² identisch sind; oder
R¹ und R² verbunden sind, um einen heterocyclischen 5- oder 6-gliedrigen Ring zu bilden, der 2 Heteroatome umfasst, ausgewählt aus N und O, der optional mit einer einzelnen funktionellen Gruppe, ausgewählt aus -C₁-C₄-Alkyl, substituiert ist, wobei der heterocyclische Ring vorzugsweise mit einer einzelnen Methylgruppe substituiert ist; oder die Dithiocarbaminsäuren oder Salze davon der Formel (I) ausgewählt sind aus der Gruppe bestehend aus den Verbindungen 1,3-18, 20-Säure, wie in Anspruch 8 definiert, oder einem Salz davon.

20. Dithiocarbaminsäure oder ein Salz davon, die/das folgendes ist:
• Verbindung 3 Säure (2,3-Dihydroxypropylcarbamodithiosäure) wie in der folgenden Tabelle definiert oder ein Salz davon,
• Verbindung 7 Säure (2-Amino-2-methyl-1,3-propandiolcarbamodithiosäure) wie in der folgenden Tabelle definiert oder ein Salz davon, oder
• Verbindung 1 Säure (Phenyl(2-aminoethyl)carbamodithiosäure) gemäß der Definition in der folgenden Tabelle oder ein Salz davon:
| **Verbindungsname** | **Verbindungsstruktur** | **CAS** | **Systematischer Name** |
|---|---|---|---|
| 3-Amino-1,2-propandioldithiocarbaminsäure **(Verbindung 3 Säure)** | 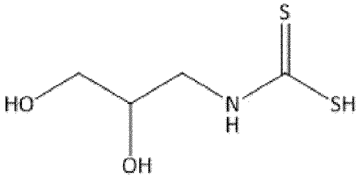 | NA | 2,3-Dihydroxypropylcarbamodithiosäure |
| 2-Amino-2-methyl-1,3-propandioldithiocarbaminsäure **(Verbindung 7 Säure)** | 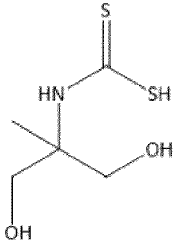 | NA | 2-Amino-2-methyl-1,3-propandiolcarbamodithiosäure |
| N-Phenylethylendiamindithiocarbaminsäure **(Verbindung 11 Säure)** | 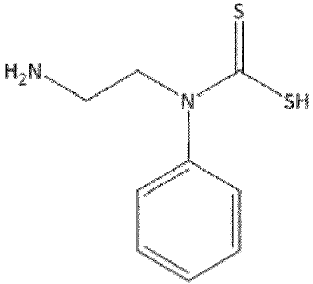 | NA | Phenyl(2-aminoethyl)-carbamodithiosäure |

21. Verwendung der Dithiocarbaminsäure oder eines Salzes davon der Formel (I) von Anspruch 20 in der Rückgewinnung, vorzugsweise der Schaumflotationsrückgewinnung, von Mineralien.

## Revendications

1. Procédé de récupération d'un premier minéral, comprenant les étapes consistant à :
(a) fournir une pulpe comprenant des solides et de l'eau, dans lequel les solides comprennent le premier minéral ;
(b) ajouter un premier déprimant à la pulpe ;
(c) soumettre la pulpe à un procédé de flottation par mousse pour produire une mousse comprenant le premier minéral ; et
(d) récupérer la mousse ;
dans lequel le premier minéral est un minéral sulfuré, et le premier déprimant consiste en un ou plusieurs acides dithiocarbamiques ou sels de ceux-ci de formule (I) 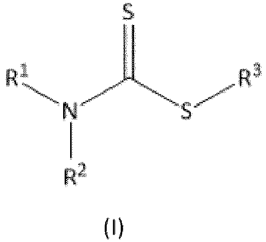
dans laquelle
R¹ représente un premier substituant présentant de 1 à 8 atomes de carbone et comprenant au moins un groupe fonctionnel choisi parmi des alcools, des amines, des éthers, des cétones, des acétals, des cétals, des aminoacétals, des éthers d'hémiaminal ou des combinaisons de ceux-ci, de préférence choisi parmi des alcools, des amines, des éthers ou des combinaisons de ceux-ci, dans laquelle R¹ comprend au plus deux groupes fonctionnels amine ;
et
R² représente H ou un second substituant présentant de 1 à 8 atomes de carbone et comprenant facultativement au moins un groupe fonctionnel choisi parmi des alcools, des amines, des éthers, des cétones, des acétals, des cétals, des aminoacétals, des éthers d'hémiaminal ou des combinaisons de ceux-ci, de préférence choisi parmi des alcools, des amines, des éthers ou des combinaisons de ceux-ci ;
R¹ et R² sont reliés pour former un cycle hétérocyclique de 3 à 7 chaînons comprenant au moins 2 hétéroatomes, qui est facultativement substitué par un, deux ou trois groupes fonctionnels choisis parmi -OH, alkyle en C₁-C₄, alcényle en C₂-C₄, hydroxyalkyle en C₁-C₄, -NR^{a}R^{b} et des combinaisons de ceux-ci, dans laquelle R^{a} et R^{b} sont choisis indépendamment parmi H et alkyle en C₁-C₄ ;
et
R³ représente de l'hydrogène ou un cation ;
à la condition que lorsque R¹ ne comprend pas d'autres groupes fonctionnels que des alcools, le procédé ne comprend pas l'utilisation d'un second déprimant choisi parmi des polymères comprenant une fonction allyl thiourée et un groupe acrylamide hydrophile ;
**caractérisé en ce que** la récupération de la mousse à l'étape (d) fournit une seconde pulpe comprenant le premier minéral qui présente une concentration accrue du premier minéral sur la base du poids sec, par comparaison à la pulpe fournie à l'étape (a).

2. Procédé de récupération d'un premier minéral, comprenant les étapes consistant à :
(a) fournir une pulpe comprenant des solides et de l'eau, dans lequel les solides comprennent le premier minéral ;
(b) ajouter un premier déprimant à la pulpe ;
(c) soumettre la pulpe à un procédé de flottation par mousse pour produire une mousse comprenant le premier minéral ; et
(d) récupérer la mousse ;
**caractérisé en ce que** le premier minéral est un minéral sulfuré, et le premier déprimant consiste en un ou plusieurs acides dithiocarbamiques ou sels de ceux-ci de formule (I) 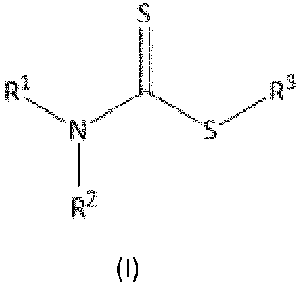
dans laquelle
R¹ représente un premier substituant présentant de 1 à 8 atomes de carbone et comprenant au moins un groupe fonctionnel choisi parmi des alcools, des amines, des éthers, des cétones, des acétals, des cétals, des aminoacétals, des éthers d'hémiaminal ou des combinaisons de ceux-ci, de préférence choisi parmi des alcools, des amines, des éthers ou des combinaisons de ceux-ci, dans laquelle R¹ comprend au plus deux groupes fonctionnels amine ;
et
R² représente H ou un second substituant présentant de 1 à 8 atomes de carbone et comprenant facultativement au moins un groupe fonctionnel choisi parmi des alcools, des amines, des éthers, des cétones, des acétals, des cétals, des aminoacétals, des éthers d'hémiaminal ou des combinaisons de ceux-ci, de préférence choisi parmi des alcools, des amines, des éthers ou des combinaisons de ceux-ci ;
R¹ et R² sont reliés pour former un cycle hétérocyclique de 3 à 7 chaînons comprenant au moins 2 hétéroatomes, qui est facultativement substitué par un, deux ou trois groupes fonctionnels choisis parmi -OH, alkyle en C₁-C₄, alcényle en C₂-C₄, hydroxyalkyle en C₁-C₄, -NR^{a}R^{b} et des combinaisons de ceux-ci, dans laquelle R^{a} et R^{b} sont choisis indépendamment parmi H et alkyle en C₁-C₄ ;
et
R³ représente de l'hydrogène ou un cation ;
à la condition que lorsque R¹ ne comprend pas d'autres groupes fonctionnels que des alcools, le procédé ne comprend pas l'utilisation d'un second déprimant choisi parmi les polymères comprenant une fonction allyl thiourée et un groupe acrylamide hydrophile ;
à la condition que le premier déprimant ne soit pas l'acide N-(2-aminoéthyl)dithiocarbamique ou un sel de celui-ci.

3. Procédé selon la revendication 1 ou 2, dans lequel R1 représente un premier substituant présentant de 2 à 5 atomes de carbone et comprenant un ou deux groupes fonctionnels choisis parmi des amines, des éthers ou des combinaisons de ceux-ci.

4. Procédé selon l'une quelconque des revendications 1 à 3, dans lequel
• R¹ représente un premier substituant présentant de 2 à 5 atomes de carbone et comprenant un ou deux alcools ; ou
• R¹ représente un premier substituant présentant de 2 à 5 atomes de carbone et comprenant une amine secondaire ou tertiaire, de préférence R¹ représente un premier substituant présentant de 4 à 5 atomes de carbone et comprenant une amine tertiaire.

5. Procédé selon l'une quelconque des revendications 1 à 4, de préférence selon la revendication 4, dans lequel
• R1 et R2 sont identiques ; ou
• R2 représente H, un phényle ou un alkyle en C1-C5, de préférence R2 représente H, un phényle ou un alkyle en C1-C2.

6. Procédé selon la revendication 1 ou 2, dans lequel R1 et R2 sont reliés pour former un cycle hétérocyclique de 5 ou 6 chaînons comprenant 2 hétéroatomes choisis parmi N et O, qui est facultativement substitué par un groupe fonctionnel unique choisi parmi alkyle en C1-C4, de préférence le cycle hétérocyclique est substitué par un groupe méthyle unique.

7. Procédé selon la revendication 1, dans lequel les acides dithiocarbamiques ou sels de ceux-ci de formule (I) sont choisis dans le groupe constitué par les acides de composé 1, 3 à 20 tels que définis dans le tableau suivant ou un sel de ceux-ci, de préférence les acides de composé 1, 3 à 6, 8 à 10, 12 à 20 tels que définis dans le tableau suivant, ou un sel de ceux-ci, plus préférablement les acides de composé 1, 3, 8, 9 tels que définis dans le tableau suivant, ou un sel de ceux-ci :
| **Nom du composé** | **Structure de composé** | **CAS** | **Nom systématique** |
|---|---|---|---|
| Acide dithiocarbamique de diéthanolamine **(acide de composé 1)** | 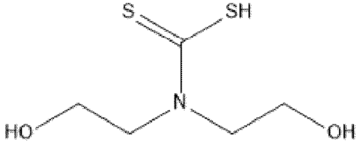 | 1528-72-9 | Acide bis(2-hydroxyétyl)carba modithioïque |
| Acide dithiocarbamique de 3-amino-1,2-propanediol **(acide de composé 3)** | 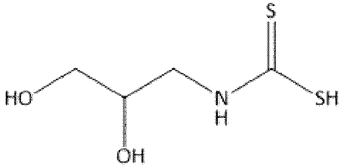 | NA | Acide 2,3-dihydroxypropylca rbamodithioïque |
| Acide dithiocarbamique de 2-(méthylamino)étha nol **(acide de composé 4)** | 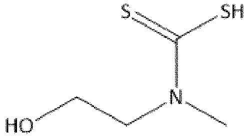 | 91308-51-9 | acide(2-hydroxyéthyl)carba modithioïque |
| Acide dithiocarbamique de bis(2-hydroxypropyl)ami ne **(acide de composé 5)** | 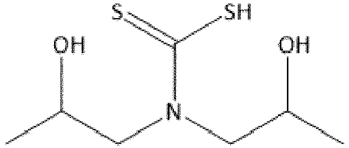 | 144796-43-0 | Acide bis(2-hydroxypropyl)car bamodithioïque |
| Acide dithiocarbamique de bis(2-méthoxyéthyl)amin e **(acide de composé 6)** | 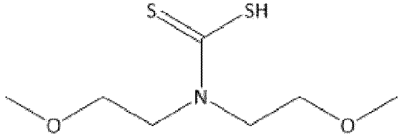 | 395653-20-0 | Acide bis(2-méthoxyéthyl)carb amodithioïque |
| Acide dithiocarbamique de 2-amino-2-méthyl-1,3,propanediol **(ac ide de composé 7)** | 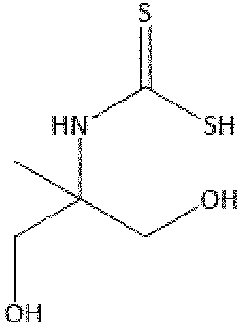 | NA | Acide carbamodhithioïqu e de 2-amino-2-méthyl-1,3,propanediol |
| Acide dithiocarbamique de morpholine **(acide de composé 8)** | 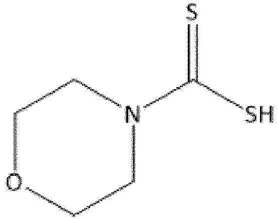 | 3581-30-4 | Acide 4-Morpholinecarbodi thioïque |
| Acide dithiocarbamique deN-méthylpipérazine ( **acide de composé 9)** | 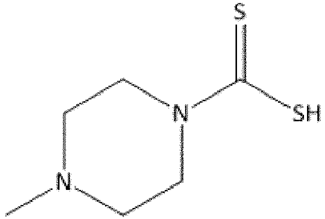 | 5430-77-3 | Acide 4-méthyl-1-pipérazinecarbodit hioïque |
| Acide dithiocarbamique de N-(2-hydroxyéthyl)anili ne **(acide de composé 10)** | 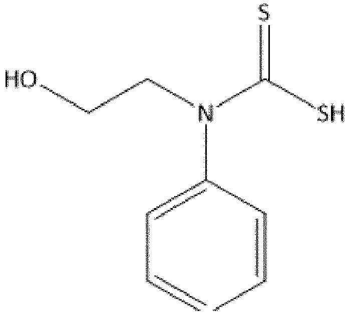 | 1006655-77-1 | Acide phényl(2-hydroxyéthyl)carba modithioïque |
| Acide dithiocarbamique de N-phényléthylènedia mine **(acide de composé 11)** | 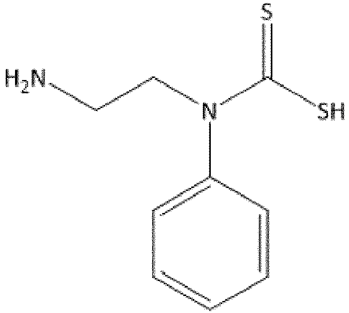 | NA | Acide phényl(2-aminoéthyl)carbam odithioïque |
| Acide dithiocarbamique de (2-méthoxyéthyl)méth ylamine **(acide de composé 12)** | 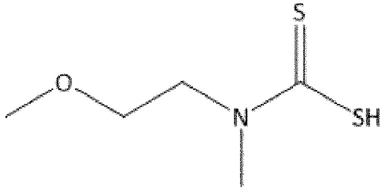 | 884739-17-7 | Acide (2-méthoxyéthyl)carb amodithioïque |
| Acide dithiocarbamique de 3,3'-iminobis(N,N-diméthylpropylami ne) **(acide de composé 13)** | 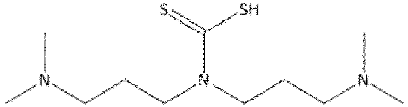 | 1161069-75-5 | Acide *Bis*[(3-diméthylamino)pro pyl] carbamodithioï que |
| Acide dithiocarbamique de 2-(éthylamino)éthano 1 **(acide de composé 14)** | 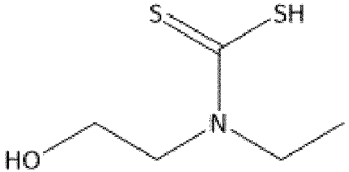 | 91308-52-0 | Acide éthyl(2-hydroxyéthyl)carba modithioïque |
| Acide dithiocarbamique de 2-(isopropylamino)ét hanol **(acide de composé 15)** | 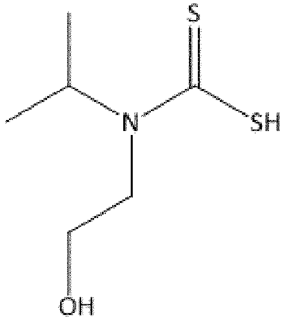 | 1442373-54-7 | Acide isopropyl(2-hydroxyéthyl)carba modithioïque |
| Acide dithiocarbamique d'éthanolamine **(ac ide de composé 16)** | 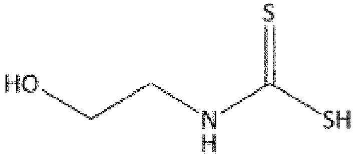 | 59333-68-5 | Acide 2-hydroxyéthylcarba modithioïque |
| Acide dithiocarbamique de N,N'-Diméthyléthyneled iamine de sodium **(acide de composé 17)** | 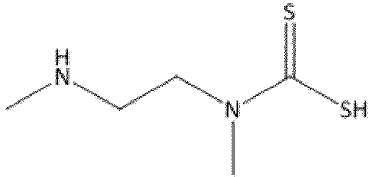 | 58708-60-4 | Acide méthyl (2-N-méthylaminoéthyl)carbam odithioïque |
| Acide dithiocarbamique de pipérazine **(acide de composé 18)** | 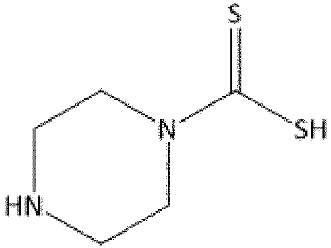 | 99-00-3 | Acide 1-pipérazinecarbodit hioïque |
| Acide dithiocarbamique d'éthylènediamine **(acide de composé 19)** | 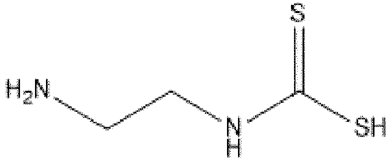 | 20950-84-9 | Acide (2-aminoéthyl)carbam odithioïque |
| Acide dithiocarbamique d'imidazole **(acide de composé 20)** | 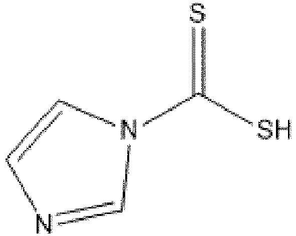 | 732930-06-1 | Acide 1H-imidazole-1-carbodithioïque |

8. Procédé selon la revendication 2, dans lequel les acides dithiocarbamiques ou sels de ceux-ci de formule (I) sont choisis dans le groupe constitué par les acides de composé 1, 3 à 18, 20 tels que définis dans le tableau suivant ou un sel de ceux-ci, de préférence acides de composé 1, 3 à 6, 8 à 10, 12 à 18, 20 tels que définis dans le tableau suivant, ou un sel de ceux-ci, plus préférablement les acides de composé 1, 3, 8, 9 tels que définis dans le tableau suivant, ou un sel de ceux-ci :
| **Nom du composé** | **Structure de composé** | **CAS** | **Nom systématique** |
|---|---|---|---|
| Acide dithiocarbamique de diéthanolamine **(ac ide de composé 1)** | 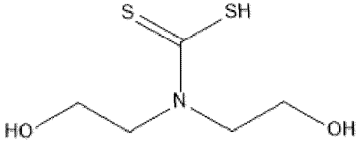 | 1528-72-9 | Acide bis(2-hydroxyétyl)carba modithioïque |
| Acide dithiocarbamique de 3-amino-1,2-propanediol **(acide de composé 3)** | 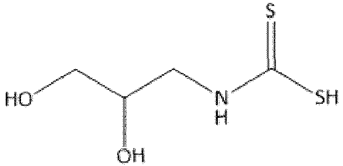 | NA | Acide 2,3-dihydroxypropylca rbamodithioïque |
| Acide dithiocarbamique de 2-(méthylamino)étha nol **(acide de composé 4)** | 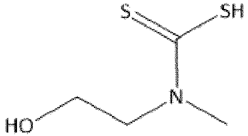 | 91308-51-9 | acide(2-hydroxyéthyl)carba modithioïque |
| Acide dithiocarbamique de bis(2-hydroxypropyl)ami ne **(acide de composé 5)** | 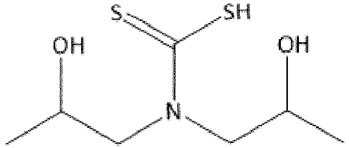 | 144796-43-0 | Acide bis(2-hydroxypropyl)car bamodithioïque |
| Acide dithiocarbamique de bis(2-méthoxyéthyl)amin e **(acide de composé 6)** | 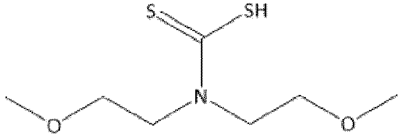 | 395653-20-0 | Acide bis(2-méthoxyéthyl)carb amodithioïque |
| Acide dithiocarbamique de 2-amino-2-méthyl-1,3,propanediol **(ac ide de composé 7)** | 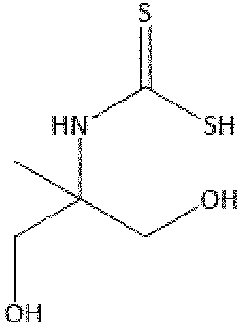 | NA | Acide carbamodhithioïqu e de 2-amino-2-méthyl-1,3,propanediol |
| Acide dithiocarbamique de morpholine **(acide de composé 8)** | 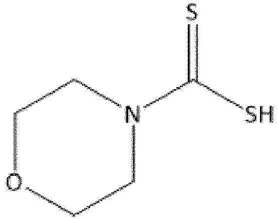 | 3581-30-4 | Acide 4-Morpholinecarbodi thioïque |
| Acide dithiocarbamique deN-méthylpipérazine ( **acide de composé 9**) | 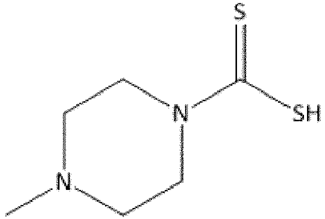 | 5430-77-3 | Acide 4-méthyl-1-pipérazinecarbodit hioïque |
| Acide dithiocarbamique de N-(2-hydroxyéthyl)anili ne **(acide de composé 10)** | 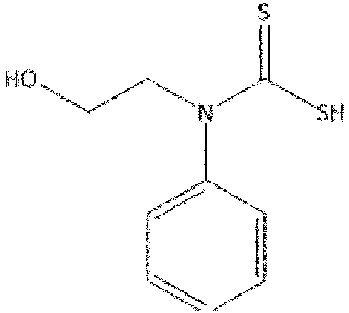 | 1006655-77-1 | Acide phényl(2-hydroxyéthyl)carba modithioïque |
| Acide dithiocarbamique de N-phényléthylènedia mine **(acide de composé 11)** | 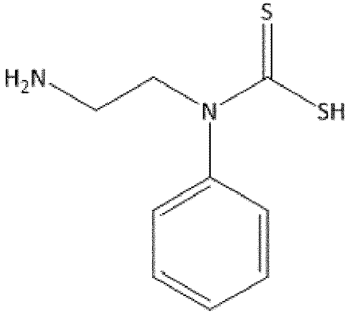 | NA | Acide phényl(2-aminoéthyl)carbam odithioïque |
| Acide dithiocarbamique de (2-méthoxyéthyl)méth ylamine **(acide de composé 12)** | 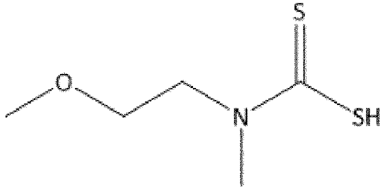 | 884739-17-7 | Acide (2-méthoxyéthyl)carb amodithioïque |
| Acide dithiocarbamique de 3,3'-iminobis(N,N-diméthylpropylami ne) **(acide de composé 13)** | 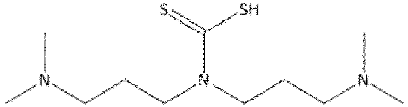 | 1161069-75-5 | Acide *Bis*[(3-diméthylamino)pro pyl] carbamodithioï que |
| Acide dithiocarbamique de 2-(éthylamino)éthano 1 **(acide de composé 14)** | 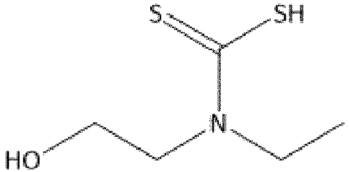 | 91308-52-0 | Acide éthyl(2-hydroxyéthyl)carba modithioïque |
| Acide dithiocarbamique de 2-(isopropylamino)ét hanol **(acide de composé 15)** | 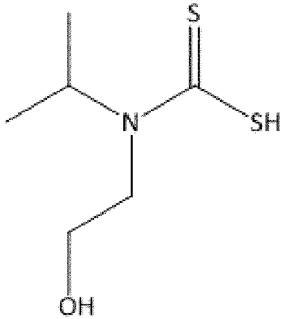 | 1442373-54-7 | Acide isopropyl(2-hydroxyéthyl)carba modithioïque |
| Acide dithiocarbamique d'éthanolamine **(ac ide de composé 16)** | 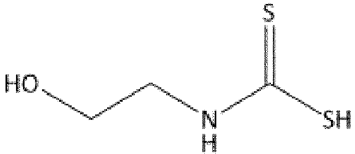 | 59333-68-5 | Acide 2-hydroxyéthylcarba modithioïque |
| Acide dithiocarbamique de N,N'-Diméthyléthyneled iamine de sodium **(acide de composé 17)** | 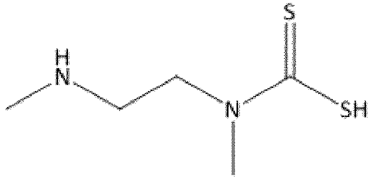 | 58708-60-4 | Acide méthyl (2-N-méthylaminoéthyl)carbam odithioïque |
| Acide dithiocarbamique de pipérazine **(acide de composé 18**) | 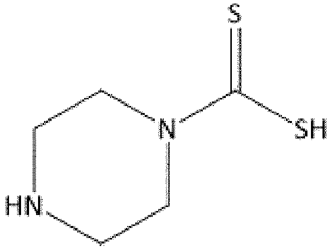 | 99-00-3 | Acide 1-pipérazinecarbodit hioïque |
| Acide dithiocarbamique d'éthylènediamine **(acide de composé 19)** | 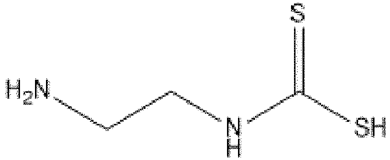 | 20950-84-9 | Acide (2-aminoéthyl)carbam odithioïque |
| Acide dithiocarbamique d'imidazole **(acide de composé 20)** | 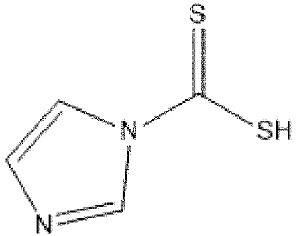 | 732930-06-1 | Acide 1H-imidazole-1-carbodithioïque |

9. Procédé selon l'une quelconque des revendications 1 à 8, dans lequel le premier minéral est un minéral de sulfure de molybdène, de préférence le premier minéral est de la molybdénite, et dans lequel les solides comprenant le premier minéral comprennent en outre un second minéral, et le procédé comprend un effet déprimant sur le second minéral dans la pulpe.

10. Procédé selon la revendication 9, dans lequel le second minéral est un minéral sulfuré, de préférence un minéral sulfuré choisi dans le groupe consistant en Chalcopyrite, Chalcocite, Covellite, Pyrite, Bornite, Sphalérite, et des combinaisons de celles-ci, de préférence Chalcopyrite.

11. Procédé selon l'une quelconque des revendications 1 à 10, de préférence selon la revendication 10, dans lequel la pulpe fournie à l'étape (a) comprend en outre un collecteur, de préférence un collecteur choisi dans le groupe comprenant des xanthates, des formates de xanthogène, des thiourées, des thionocarbamates, des (di)thiophosphates, des dithiophosphinates, des N-alcoxycarbonyl dithiocarbamates, des dialkyldithiocarbamates, des mercaptobenzothioazoles, des nitriles et des combinaisons de ceux-ci.

12. Procédé selon l'une quelconque des revendications 1 à 11, comprenant en outre l'étape consistant à :
(b2) ajouter un second déprimant à la pulpe fournie à l'étape (a) ;
dans lequel l'étape (b2) peut être réalisée avant, pendant et/ou après l'étape (b) et de préférence le second déprimant est NaHS.

13. Composition comprenant le premier déprimant tel que décrit dans l'une quelconque des revendications 1, 3 à 7 et comprenant en outre un minéral sulfuré, de préférence un minéral sulfuré choisi dans le groupe consistant en Molybdénite, Chalcopyrite, Chalcocite, Covellite, Pyrite, Bornite, Sphalérite, et des combinaisons de celles-ci, de préférence choisi dans le groupe consistant en Molybdénite et Chalcopyrite ;
à condition que le premier déprimant ne soit pas l'acide N-(2-Aminoéthyl)carbamodithioïque ou un sel de celui-ci ou un composé de formule (I) dans lequel R¹ ne comprend pas d'autres groupes fonctionnels que des alcools.

14. Composition comprenant un premier déprimant et comprenant en outre un, deux ou trois des éléments suivants :
• un collecteur tel que décrit dans la revendication 11 ;
• un second déprimant tel que décrit dans la revendication 12 ; et
• un minéral ;
dans laquelle le premier déprimant consiste en un ou plusieurs acides dithiocarbamiques ou sels de ceux-ci de formule (I) tels que décrits dans la revendication 1,
dans laquelle
R¹ représente un premier substituant présentant de 2 à 5 atomes de carbone et comprenant un ou deux alcools ; ou
R¹ et R² sont identiques ; ou
R¹ et R² sont reliés pour former un cycle hétérocyclique de 5 ou 6 chaînons comprenant 2 hétéroatomes choisi parmi N et O, qui est facultativement substitué par un groupe fonctionnel unique choisi parmi alkyle en C₁-C₄, de préférence le cycle hétérocyclique est substitué par un groupe méthyle unique ; ou
les acides dithiocarbamiques ou sels de ceux-ci de formule (I) sont choisis dans le groupe constitué par les acides de composé 1, 3 à 18, 20 tels que définis dans la revendication 8 ou un sel de ceux-ci,
à condition que le premier déprimant ne soit pas un composé de formule (I) dans laquelle R¹ ne comprend pas d'autres groupes fonctionnels que des alcools.

15. Composition selon la revendication 13 ou 14, qui est une composition d'additif minier comprenant un second déprimant tel que décrit dans la revendication 12 en une quantité d'au moins 5 % en poids, sur la base du poids total de la composition d'additif, à l'exclusion de solvants,
de préférence d'au moins 10 % en poids, sur la base du poids total de la composition d'additif, à l'exclusion de solvants ;
dans lequel le premier déprimant est de préférence présent en une quantité d'au moins 5 % en poids, sur la base du poids total de la composition d'additif, à l'exclusion de solvants,
de préférence d'au moins 20 % en poids, sur la base du poids total de la composition d'additif, à l'exclusion de solvants.

16. Composition selon la revendication 13 ou 14, qui est une pulpe minérale comprenant un minéral sulfuré, de préférence un minéral sulfuré choisi dans le groupe constitué de Molybdénite, Chalcopyrite, Chalcocite, Covellite, Pyrite, Bomite, Sphalérite, et des combinaisons de celles-ci, en une quantité d'au moins 0,01 % en poids, sur la base du poids total de la pulpe minérale, de préférence d'au moins 0,1 % en poids, et comprenant en outre facultativement
• un collecteur tel que décrit dans la revendication 11 en une quantité d'au moins 10 ppm p/p, sur la base du poids total de la pulpe minérale ;
• et/ou
• un second déprimant tel que décrit dans la revendication 12 en une quantité d'au moins 10 ppm, sur la base du poids total de la pulpe minérale ;
dans lequel le premier déprimant est de préférence présent en une quantité d'au moins 10 ppm, sur la base du poids total de la pulpe minérale.

17. Utilisation d'un acide dithiocarbamique ou d'un sel de celui-ci de formule (I) tel que décrit dans l'une quelconque des revendications 1 à 8 dans la récupération, de préférence la récupération par flottation par mousse, de minéraux à condition que l'acide dithiocarbamique ou un sel de celui-ci ne soit pas l'acide N-(2-Aminoéthyl)carbamodithioïque ou un sel de celui-ci ou un composé de formule (I) dans lequel R1 ne comprend pas d'autres groupes fonctionnels que des alcools.

18. Utilisation selon la revendication 17 dans la récupération par flottation de mousse d'un minéral sulfuré, de préférence de Molybdénite,

19. Utilisation selon la revendication 17 ou 18, dans laquelle l'acide dithiocarbamique ou un sel de celui-ci est un acide dithiocarbamique ou un sel de celui-ci de formule (I) telle que décrite dans la revendication 1, dans laquelle
R¹ représente un premier substituant présentant de 2 à 5 atomes de carbone et comprenant un ou deux alcools ; ou
R¹ et R² sont identiques ; ou
R¹ et R² sont connectés pour former un cycle hétérocyclique de 5 ou 6 chaînons comprenant 2 hétéroatomes choisis parmi N et O, qui est facultativement substitué par un groupe fonctionnel unique choisi parmi alkyle en C₁-C₄, de préférence le cycle hétérocyclique est substitué par un groupe méthyle unique ; ou
les acides dithiocarbamiques ou sels de ceux-ci de formule (I) sont choisis dans le groupe constitué par les acides de composé 1, 3 à 18, 20 tels que définis dans la revendication 8 ou un sel de ceux-ci.

20. Acide dithiocarbamique ou sel de celui-ci qui est
• un acide de composé 3 (acide 2,3-dihydroxypropylcarbamodithioïque) tel que défini dans le tableau suivant ou un sel de celui-ci,
• un acide de composé 7 (acide 2-amino-2-méthyl-1,3-propanediolcarbamodithioïque) tel que défini dans le tableau suivant ou un sel de celui-ci, ou
• un acide de composé 11 (acide phényl(2-aminoéthyl)carbamodithioïque) tel que défini dans le tableau suivant ou un sel de celui-ci :
| **Nom du composé** | **Structure de composé** | **CAS** | **Nom systématique** |
|---|---|---|---|
| Acide dithiocarbamique de 3-amino-1,2-propanediol **(acide de composé 3)** | 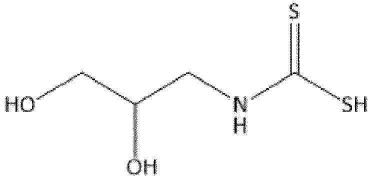 | NA | Acide 2,3-dihydroxypropylca rbamodithioïque |
| Acide dithiocarbamique de 2-amino-2-méthyl-1,3,propanediol **(acide de composé 7)** | 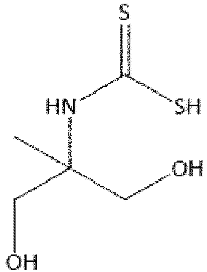 | NA | Acide carbamodhithioïqu e de 2-amino-2-méthyl-1,3,propanediol |
| Acide dithiocarbamique de N-phényléthylènediamin e **(acide de composé 11)** | 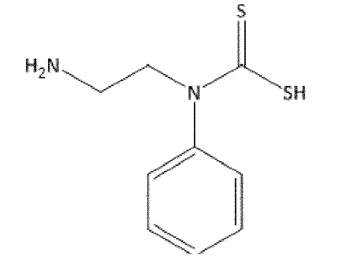 | NA | Acide phényl-(2-aminoéthyl)carbam odithioïque |

21. Utilisation de l'acide dithiocarbamique ou d'un sel de celui-ci de formule (I) de la revendication 20 dans la récupération, de préférence la récupération par flottation par mousse, de minéraux.
